# EUROPÄISCHE PATENTSCHRIFT

(11) **EP 3 634 365 B1**
(45) Veröffentlichungstag und Bekanntmachung des Hinweises auf die Patenterteilung: **10.08.2022**
(21) Anmeldenummer: 18726092.2
(22) Anmeldetag: 14.05.2018
(51) Int. Cl.: A61K 8/34, A61K 8/39, A61Q 5/10, A61K 8/86

(54) **HAARFÄRBEMITTEL MIT VERBESSERTER SALZTOLERANZ**
HAIR COLORING COMPOSITION WITH IMPROVED SALT TOLERANCE
PRODUIT DE COLORATION CAPILLAIRE DOTÉ D'UNE TOLÉRANCE AU SEL AMÉLIORÉE

(30) Priorität: 09.06.2017 DE 102017209769
(43) Veröffentlichungstag der Anmeldung: 15.04.2020
(73) Patentinhaber: Henkel AG & Co. KGaA, 40589 Düsseldorf (DE)
(72) Erfinder: MÜLLER, Burkhard, 40221 Düsseldorf (DE); BONNIN, Lucile, 40215 Düsseldorf (DE)
(86) Internationale Anmeldenummer: PCT/EP2018/062290
(87) Internationale Veröffentlichungsnummer: WO 2018/224244

(56) Entgegenhaltungen:
- WO-A1-2016/093363
- JP-A- 2005 247 742
- JP-A- 2007 217 293
- US-A1- 2015 082 554

## Beschreibung

Die vorliegende Erfindung liegt auf dem Gebiet der Kosmetik. Gegenstand der vorliegenden Erfindung ist eine Mehrkomponenten-Verpackungseinheit zur Färbung von keratinischen Fasern, in welcher ein Mittel (A) getrennt konfektioniert neben einer Oxidationsmittelzubereitung (B) vorliegt, wobei die Mittel (A) neben Farbstoffen und Alkalisierungsmitteln eine bestimmte Kombination aus (a) hoch ethoxylierten Fettalkoholen, (b) niedrig ethoxylierten Fettalkoholen, (c) Mono-GlycerinFettsäureestern und (d) Fettalkoholen enthält. Kennzeichnend für diese Mittel (A) ist, dass sie die vorgenannten Inhaltsstoffe (a) bis (d) in ganz speziellen Gewichtsverhältnissen zueinander enthalten.

Die Anwendung von Emulsionen ist in der Kosmetik weit verbreitet. In einer Emulsion liegt ein fein verteiltes Gemisch zweier Flüssigkeiten, wie beispielsweise Fettkörper (Öle, Fettalkohole, Kohlenwasserstoffe oder auch Fettsäuretriglyceride) und Wasser vor. Eine Theorie zu Emulsionen ist, dass eine der Flüssigkeiten (Phase) kleine Tröpfchen bildet, die verteilt in der anderen Flüssigkeit (Phase) vorliegen. Die Phase, welche die Tröpfchen bildet, wird als innere Phase oder auch disperse Phase bezeichnet. Die Phase, in der die Tröpfchen schwimmen, wird die äußere Phase oder auch die kontinuierliche Phase genannt.

Bei Emulsionen, die eine Wasserphase und eine Ölphase umfassen, werden Öl-in-Wasser Emulsionen (O/W-Emulsionen) und Wasser-in-ÖI-Emulsionen (W/O-Emulsionen) unterschieden. Klassische O/W-Emulsionen werden in der Literatur oft als Öltröpfchen beschrieben, die in der kontinuierlichen Wasserphase dispergiert sind und an der Grenzfläche beider Phasen durch Tenside oder Emulgatoren stabilisiert sind. Letztere formen einen Film um die Öltröpfchen und sind so in der Lage, die Oberflächenspannung herabzusetzen. In komplexen kosmetischen Formulierungen wird jedoch in der Regel eine Vielzahl verschiedener Inhaltstoffe eingesetzt, wodurch kompliziertere mehrphasige Systeme entstehen.

Viele Mittel zum Färben von keratinischen Fasern bzw. menschlichen Haaren liegen in Form von Emulsionen vor. Eine derartige Emulsion kann dann als stabil bezeichnet werden, wenn die Fusion der Tröpfchen durch eine ausreichend hohe Energiebarriere verhindert werden kann. Generell wird diese Energiebarriere durch den Film gebildet, den der oder die Emulgatoren auf der Oberfläche des jeweiligen Tröpfchens ausbilden. Ist die Emulsion instabil, so bricht sie und trennt sich in Öl- und Wasserphase auf. Kosmetische Produkte, insbesondere Haarfärbemittel, müssen oft Lagerzeiträume von vielen Monaten überstehen. Es ist eine wesentliche Qualitätsanforderung, dass die emulsionsförmigen Färbmittel über den gesamten Lagerzeitraum stabil bleiben und sich nicht während der Lagerung trennen.

Zur Erzeugung von permanenten, intensiven Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch intensive, hervorragende, lang anhaltende Färbeergebnisse aus. Für natürlich wirkende Färbungen kann eine Mischung aus einer größeren Zahl von Oxidationsfarbstoffvorprodukten eingesetzt werden, wobei in vielen Fällen weiterhin zusätzlich direktziehende Farbstoffe zur Nuancierung verwendet werden.

Oxidationsfarbstoffvorprodukte vom Entwicklertyp basieren typischerweise auf der Grundstruktur des p-Phenylendiamins, des p-Aminophenols oder von heterozyklischen Di- oder Poly-Aminoverbindungen. Substanzen dieses Typs sind gegenüber Luftsauerstoff höchst empfindlich und werden zur Stabilisierung meist in Form ihrer physiologisch verträglichen Salze eingesetzt, d.h. die in den Substanzen vorhandenen Aminogruppen werden - ganz oder teilweise - in Ammoniumgruppen überführt und durch Gegenionen (Chloride, Bromide, Hydrogensulfate oder auch Sulfate) neutralisiert. Wünscht sich der Anwender die Färbung seiner Haare in einem besonders dunklen Farbton, wie beispielsweise einer dunkelbraunen oder schwarzen Nuance, so greift er zu einem entsprechenden Färbemittel mit besonders hohem Farbstoffgehalt. Bedingt durch den hohen Gehalt an Oxidationsfarbstoffvorprodukten ist auch der entsprechende Salzgehalt in diesen Mitteln sehr hoch.

Emulsionen wie beispielsweise O/W-Emulsionen reagieren gegenüber einer Erhöhung ihres Salzgehaltes oft sehr empfindlich. Die Gefahr, dass eine Emulsion bzw. ein Färbemittel sich trennt und als nicht lagerstabil erweist, ist bei Nuancen mit hohem Farbstoffgehalt daher besonders groß.

Weiterhin umfasst der kommerzielle Vertrieb einer bestimmten Haarfärbemarke in der Regel ein bestimmtes Nuancen-Portfolio, aus dem der Anwender seine gewünschte Farbe auswählen kann.

Üblicherweise wird innerhalb dieses Portfolios eine für alle Nuancen gleiche Basiscreme eingesetzt, in welcher abhängig von dem jeweiligen Farbton verschiedene Mengen an Oxidationsfarbstoffvorprodukten eingesetzt werden. Je dunkler die Nuance ist, desto höher ist der Gehalt an Oxidationsfarbstoffen und desto höher ist damit auch der Salzgehalt in der Emulsion. Um innerhalb des Portfolios die gleiche Basis-Creme verwenden zu können, ist es von zentraler Bedeutung, dass diese Creme sowohl bei Einsatz von niedrigen als auch bei Einsatz von höhen Farbstoff-Konzentrationen eine gleichbleibende und hohe Stabilität besitzt. Um die Produktionsprozesse zu vereinfachen, sollte unabhängig von der jeweils gewählten Farbstoffmenge auch die Viskosität der Farbcreme gleich bleiben.

Die Aufgabe der vorliegenden Erfindung lag daher in der Bereitstellung von Färbemitteln (insbesondere Farbcremes zur oxidativen Farbveränderung) in Emulsionsform, welche eine verbesserte Stabilität mit erhöhter Salztoleranz und optimierter Viskositäts-Stabilität besitzen. Da die Farbcremes in der Regel alkalisch eingestellt sind, soll diese Stabilisierung auch unter alkalischen Bedingungen gewährleistet sein.

Die grundlegende Voraussetzung hierbei war, dass die Färbemittel die oben genannten Verbesserungen aufzeigen sollten, ohne Einbußen im Hinblick auf die weiteren anwendungstechnischen Eigenschaften, zu erleiden. Die Farbintensitäten, Waschechtheiten und Lichtechtheiten dieser Mittel sollten sich gegenüber den aus dem Stand der Technik bekannten Mitteln also nicht verschlechtern und optimaler Weise weiter verbessern.

Gegenstand von JP 2007-217293 A ist ein auf zwei Komponenten basierendes Mittel zur Färbung bzw. Bleiche von Haaren, wobei die erste Komponente 2 bis 18 Gew.-% Tensid, 0,1 bis 12 Gew.-% Öl(e) und 0,1 bis 12 Gew.-% Wasserstoffperoxid enthält.

Überraschenderweise hat sich nun herausgestellt, dass die Salztoleranz einer alkalischen Farbcreme stark verbessert werden konnte, wenn eine ganz bestimmte Kombination von nichtionischen, lipophilen und hydrophilen Emulgatoren in bestimmten Mengenbereichen zueinander eingesetzt wurde. So wurden besonders stabile und gegenüber schwankenden Salzmengen unempfindliche Emulsionen erhalten, wenn in den Emulsionen (a) mindestens ein hoch ethoxylierter C₁₆-C₁₈-Fettalkohol (mit 30 bis 100 EO-Gruppen), (b) mindestens ein niedrig ethoxylierter C₁₆- C₁₈-Fettalkohol (mit 1 bis 5 EO-Gruppen), (c) gegebenenfalls mindestens ein Monoester aus Glycerin und einer C₁₄-C₂₀-Fettsäure und (d) mindestens ein C₁₆-C₁₈-Fettalkohol eingesetzt wurde.

Als wesentlich haben sich hierbei die Gewichtsverhältnisse erwiesen, in denen die Bestandteile (a) bis (d) in der alkalischen Farbcreme (A) vorhanden sind. So ist eine erste wichtige Vorraussetzung, die hoch ethoxylierten C₁₆-C₁₈-Fettalkohole (a) im Vergleich zu den niedrig ethoxylierten C₁₆-C₁₈-Fettalkoholen (b) in einem 10- bis 20-fachen Gewichtsüberschuss im Mittel einzusetzen, d.h. im erfindungsgemäßen Mittel (A) liegt das Gewichtsverhältnis (a)/(b) bei einem Wert von 10 bis 20.

Eine zweite wichtige Voraussetzung besteht darin, dass die liphophilen Emulgatorbestandteile (c) plus (d) im Vergleich zu den ethoxylierten Fettalkoholen (a) plus (b) ebenfalls in einem Überschuss eingesetzt werden. Hierbei ist die Summe der im Mittel eingesetzten Gewichtsmengen aus [(c)+(d)] im Vergleich zur Summe der im Mittel eingesetzten Gewichtsmengen aus [(a)+(b)] um den Faktor 2 bis 5-fachen höher.

Ein erster Gegenstand der vorliegenden Erfindung ist eine Mehrkomponenten-Verpackungseinheit (Kit-of-Parts) zum oxidativen Färben von keratinischen Fasern, insbesondere menschlichen Haaren, umfassend getrennt voneinander konfektioniert
- einen Container (I) enthaltend ein kosmetisches Mittel (A) und
- einen Container (II) enthaltend ein kosmetisches Mittel (B),
wobei
- das Mittel (A) in Container (I) ein Mittel zur Färbung von keratinischen Fasern ist, enthaltend in einem wässrigen Träger
   (a) einen oder mehrere hoch ethoxylierte C₁₆-C₁₈-Fettalkohole mit 30 bis 100 Ethoxy- Gruppen,
   (b) einen oder mehrere niedrig ethoxylierte C₁₆-C₁₈-Fettalkohole mit 1 bis 5 Ethoxygruppen,
   (c) einen oder mehrere Monoester aus Glycerin und einer C₁₄-C₂₀- Fettsäure,
   (d) einen oder mehrere C₁₆-C₁₈-Fettalkohole,
   (e) ein oder mehrere Alkalisierungsmittel und
   (f) ein oder mehrere Oxidationsfarbstoffvorprodukte, wobei
- das Gewichtsverhältnis aus allen im Mittel enthaltenen hoch ethoxylierten C₁₆-C₁₈-Fettalkoholen (a) zu allen im Mittel enthaltenen niedrig ethoxylierten C₁₆-C₁₈-Fettalkoholen (b), d.h. das Gewichtsverhältnis (a)/(b), bei einem Wert von 10 bis 20 liegt und
- das Gewichtsverhältnis aus der Summe aller im Mittel enthaltenen Bestandteile [(c)+(d)] zur Summe aller im Mittel enthaltenden ethoxylierten Fettalkohole [(a)+(b)], d.h. das Gewichtsverhältnis [(c)+(d)]/[(a)+(b)], bei einem Wert von 2 bis 5 liegt, und
- das Mittel (B) in Container (II) eine Oxidationsmittelzubereitung (B) ist, die Wasserstoff- peroxid enthält.

### Keratinische Fasern

Bei dem Mittel handelt es sich um ein Mittel zur Färbung von keratinischen Fasern, insbesondere menschlichen Haaren. Besonders bevorzugt wird das Mittel zum oxidativen Färben von keratinischen Fasern, insbesondere menschlichen Haaren, eingesetzt.

Unter keratinischen Fasern sind Wolle, Pelze, Federn und insbesondere menschliche Haare zu verstehen. Die erfindungsgemäßen Mittel zur oxidativen Farbveränderung können prinzipiell aber auch zur Farbveränderung anderer Naturfasern, wie z. B. Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose verwendet werden.

### Mittel (A) zu Färbung von keratinischen Fasern

In der erfindungsgemäßen Mehrkomponenten-Verpackungseinheit wird das erfindungsgemäße Mittel (A) zur oxidativen Färbung von keratinischen Fasern eingesetzt. Mit den Bestandteilen (e) und (f) enthält das Mittel (A) Alkaliserungsmittel und Oxidationsfarbstoffvorprodukte. Damit handelt es sich bei dem Mittel (A) um eine alkalisch eingestellte Farbcreme, die vor der Anwendung mit einem Oxidationsmittel vermischt und dann als anwendungsbereites Mittel auf die Keratinfasern (Haare) appliziert wird.

Das Mittel (A) enthält als erfindungswesentliche Bestandteile die Komponenten (a) und (b) und (c) und (d) und (e) und (f). Weiterhin kann die Komponente (c) als optionaler Bestandteil im Mittel enthalten sein.

### Wässriger Träger

Das erfindungsgemäße Mittel (A) enthält die Bestandteile (a) bis (f) in einem wässrigen Träger. Die Bestandteile (a) und (b) stellen Fettalkohole mit hohem bzw. niedrigem Ethoxylierungsgrad dar. Der Bestandteil (c) ist ein Mono-Fettsäure-Glycerid, und bei dem Bestandteil (d) handelt es sich um C₁₆-C₁₈-Fettalkohole. Damit sind (a) bis (d) nicht- ionische Verbindungen mit Emulgator-Eigenschaften, die sich hinsichtlich ihrer Lipophilie bzw. Hydrophilie unterscheiden. Zusammen bilden die Bestandteile eine Emulsion aus, bei der es sich um eine Öl-in-Wasser-Emulsion handelt. Wasser ist hierbei die äußere Phase oder auch die kontinuierliche Phase.

Besonders bevorzugt enthält das erfindungsgemäße Mittel (A) - bezogen auf sein Gesamtgewicht - 50 bis 95 Gew.-%, bevorzugt 55 bis 90 Gew.-%, weiter bevorzugt 60 bis 85 Gew.-% und ganz besonders bevorzugt 65 bis 80 Gew.-% Wasser.

### (a) Hoch ethoxylierte Fettalkohole

Als ersten erfindungswesentlichen Bestandteil (a) enthalten die erfindungsgemäßen Mittel (A) einen oder mehrere hoch ethoxylierte C₁₆-C₁₈-Fettalkohole mit 30 bis 100 Ethoxy-Gruppen. Als Ethoxy- Gruppe wird in diesem Fall die Struktureinheit -CH₂-CH₂-O- bezeichnet, die alternativ auch als Ethylenoxid-Einheit bezeichnet werden kann.

Die Molmenge an Ethylenoxid, die pro Mol Fettalkohol eingesetzt wurde, bezeichnet dabei den Ethoxylierungsgrad. Unter hoch ethoxylierten Fettalkohlen werden demnach Fettalkohole verstanden, die mit mindestens 30 und höchstens 100 Ethoxygruppen ethoxyliert wurden (d.h. jedes mol Fettalkohol wurde mit 30 mol bis 100 mol Ethylenoxid ethoxyliert).

Mit anderen Worten ist ein erfindungsgemäßes Mittel (A) dadurch gekennzeichnet, dass es (a) ein oder mehrere ethoxylierte C₁₆-C₁₈-Fettalkohole mit einem Ethoxylierungsgrad von 30 bis 100 enthält.

Bei C₁₆-C₁₈-Fettalkoholen handelt es sich erfindungsgemäß um lineare oder verzweigte, gesättigte oder ungesättigte Alkanole mit 16 bis 18 Kohlenstoffatomen. Ungesättigte C₁₆-C₁₈-Fettalkohole können einfach oder mehrfach ungesättigt sein. Bei einem ungesättigten Fettalkohol kann bzw. können dessen C-C-Doppelbindung(en) die Cis- oder Trans-Konfiguration aufweisen. Es ist erfindungsgemäß ebenfalls möglich, Gemische von Fettalkoholen, die durch gezielte Mischung oder auch durch Gewinnungsverfahren als solche anfallen, einzusetzen. Ein Beispiel ist Cetearylalkohol (1:1-Mischung aus C₁₆- und C₁₈-Fettalkoholen).

Besonders bevorzugt enthält das Mittel (A) als hoch ethoxylierten C₁₆-C₁₈-Fettalkohol mindestens eine Verbindung der Formel (I) 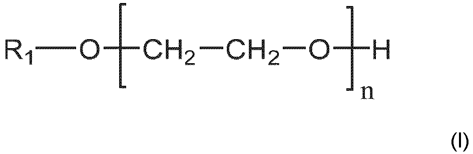 worin
R1 für eine lineare oder verzweigte, gesättigte oder ungesättigte C₁₆-C₁₈-Alkylgruppe, besonders bevorzugt für eine Cetylgruppe (C₁₆-Alkylgruppe) oder eine Stearylgruppe (C₁₈-Alkylgruppe) steht und
n für eine ganze Zahl von 30 bis 100, bevorzugt von 35 bis 90, weiter bevorzugt von 40 bis 80 und ganz besonders bevorzugt von 45 bis 70 steht.

In einer besonders bevorzugten Ausführungsform ist ein erfindungsgemäßes Mittel (A) dadurch gekennzeichnet, dass es (a) einen oder mehrere hoch ethoxylierte C₁₆-C₁₈-Fettalkohole der Formel (I) enthält, 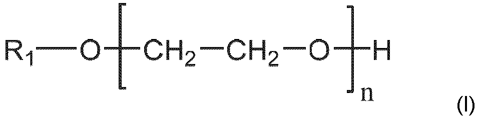 worin
R1 für eine lineare oder verzweigte, gesättigte oder ungesättigte C₁₆-C₁₈-Alkylgruppe steht und
n für eine ganze Zahl von 30 bis 100, bevorzugt von 35 bis 90, weiter bevorzugt von 40 bis 80 und ganz besonders bevorzugt von 45 bis 70 steht.

Als geeignete hoch ethoxylierte Fettalkohole der Formel (I) können beispielsweise Ceteareth-30, Steareth-30, Ceteth-30, Oleth-30, Ceteareth-50, Steareth-50, Ceteth-50, Oleth-50, Ceteareth-100, Steareth-100, Ceteth-100 und Oleth-100 genannt werden.

Ganz besonders bevorzugt werden in den erfindungsgemäßen Mitteln Ceteareth-50, Steareth-50, und/oder Ceteth-50 eingesetzt.

Der oder die hoch ethoxylierten C₁₆-C₁₈-Fettalkohole werden bevorzugt in bestimmten Mengenbereichen im erfindungsgemäßen Mittel (A) eingesetzt, wobei für alle Mengenbereiche (a) die Prämisse gilt, dass gleichzeitig die beiden Verhältnisbedingungen betreffend die Gewichtsverhältnisse (a)/(b) sowie [(c)+(d)]/[(a)+(b)] erfüllt sind.

Besonders stabile und salztolerante Emulsionen konnten erhalten werden, wenn die Mittel (A) einen oder mehrere hoch ethoxylierte C16-C18-Fettalkohole mit 30 bis 100 Ethoxy-Gruppen in einer Gesamtmenge von 2,0 bis 10,0 Gew.-%, bevorzugt von 2,0 bis 8,0 Gew.-%, weiter bevorzugt von 2,0 bis 6,0 Gew.-% und ganz besonders bevorzugt von 3,0 bis 5,0 Gew.-% enthielten. Alle vorgenannten Mengenbereiche in Gew.-% sind hierbei auf die Gesamtmenge der im Mittel (A) enthaltenen hoch ethoxylierten Fettalkohole (a) bezogen, die zum Gesamtgewicht des Mittels in Relation gesetzt wird.

In einer weiteren ganz besonders bevorzugten Ausführungsform ist ein erfindungsgemäßes Mittel (A) somit dadurch gekennzeichnet, dass es - bezogen auf das Gesamtgewicht des Mittels (A) - (a) einen oder mehrere hoch ethoxylierte C16-C18-Fettalkohole mit 30 bis 100 Ethoxy-Gruppen in einer Gesamtmenge von 2,0 bis 10,0 Gew.-%, bevorzugt von 2,0 bis 8,0 Gew.-%, weiter bevorzugt von 2,0 bis 6,0 Gew.-% und ganz besonders bevorzugt von 3,0 bis 5,0 Gew.-% enthält.

### (b) Niedrig ethoxylierte Fettalkohole

Als zweiten erfindungswesentlichen Bestandteil (b) enthalten die erfindungsgemäßen Mittel (A) einen oder mehrere niedrig ethoxylierte C₁₆-C₁₈-Fettalkohole mit 1 bis 5 Ethoxygruppen.

Unter niedrig ethoxylierten Fettalkohlen werden Fettalkohole verstanden, die mit mindestens 1 und höchstens 5 Ethoxygruppen (EO-Gruppen) ethoxyliert wurden. Als Ethoxy-Gruppe wird in diesem Fall wieder die Struktureinheit -CH₂-CH₂-O- bezeichnet, die alternativ auch als Ethylenoxid-Einheit bezeichnet werden kann.

Mit anderen Worten ist ein erfindungsgemäßes Mittel (A) dadurch gekennzeichnet, dass es (b) ein oder mehrere ethoxylierte C₁₆-C₁₈-Fettalkohole mit einem Ethoxylierungsgrad von 1 bis 5 enthält.

Wie zuvor beschrieben handelt es sich bei den C₁₆-C₁₈-Fettalkoholen erfindungsgemäß um lineare oder verzweigte, gesättigte oder ungesättigte Alkanole mit 16 bis 18 Kohlenstoffatomen. Ungesättigte C₁₆-C₁₈-Fettalkohole können einfach oder mehrfach ungesättigt sein. Bei einem ungesättigten Fettalkohol kann bzw. können dessen C-C-Doppelbindung(en) die Cis- oder Trans-Konfiguration aufweisen. Es ist erfindungsgemäß ebenfalls möglich, Gemische von Fettalkoholen, die durch gezielte Mischung oder auch durch Gewinnungsverfahren als solche anfallen, einzusetzen. Ein Beispiel ist Cetearylalkohol (1:1-Mischung aus C₁₆- und C₁₈-Fettalkoholen).

Besonders bevorzugt enthält das Mittel (A) als niedrig ethoxylierten C₁₆-C₁₈-Fettalkohol (b) mindestens eine Verbindung der Formel (II) 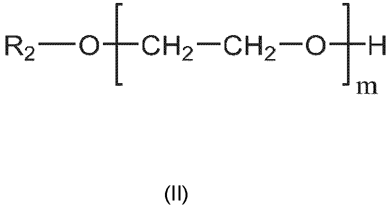 worin
R2 für eine lineare oder verzweigte, gesättigte oder ungesättigte C₁₆-C₁₈-Alkylgruppe steht und m für eine ganze Zahl von 1 bis 5, bevorzugt von 1 bis 4 und ganz besonders bevorzugt von 1 bis 3 steht.

In einer besonders bevorzugten Ausführungsform ist ein erfindungsgemäßes Mittel (A) dadurch gekennzeichnet, dass es (b) einen oder mehrere niedrig ethoxylierte C₁₆-C₁₈-Fettalkohole der Formel (II) enthält, 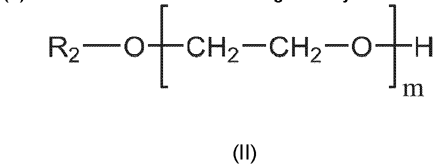 worin
R2 für eine lineare oder verzweigte, gesättigte oder ungesättigte C₁₆-C₁₈-Alkylgruppe steht und m für eine ganze Zahl von 1 bis 5, bevorzugt von 1 bis 4 und ganz besonders bevorzugt von 1 bis 3 steht.

Als geeignete niedrig ethoxylierte Fettalkohole der Formel (II) können beispielsweise Ceteareth-2, Steareth-2, Ceteth-2, Oleth-2, Ceteareth-3, Steareth-3, Ceteth-3, Oleth-3, Ceteareth-4, Steareth-4, Ceteth-4, Oleth-4, Ceteareth-5, Steareth-5, Ceteth-5 und/oder Oleth-5 genannt werden.

Ganz besonders bevorzugt werden in den erfindungsgemäßen Mitteln Ceteareth-2, Steareth-2, und/oder Ceteth-2 eingesetzt.

Um im Hinblick auf die Salzstabilität optimale Ergebnisse zu erhalten, werden auch die niedrig ethoxylierten Fettalkohole mit 1 bis 5 Ethoxy-Gruppen bevorzugt in bestimmten Mengenbereichen im erfindungsgemäßen Mittel (A) eingesetzt.

Besonders stabile und salztolerante Emulsionen konnten erhalten werden, wenn die Mittel (A) einen oder mehrere niedrig ethoxylierte C₁₆-C₁₈-Fettalkohole (b) mit 1 bis 5 Ethoxy-Gruppen in einer Gesamtmenge von 0,05 bis 1,5 Gew.-%, bevorzugt von 0,05 bis 1,25 Gew.-%, weiter bevorzugt von 0,1 bis 1,0 Gew.-% und ganz besonders bevorzugt von 0,1 bis 0,75 Gew.-% enthielten.

Auch bei diesen Mengenangaben betreffend die im Mittel enthaltene Gesamtmenge an niedrig ethxoylierten Fettalkoholen (b) gilt wieder die Voraussetzung, dass gleichzeitig die beiden Verhältnisbedingungen betreffend die Gewichtsverhältnisse (a)/(b) sowie [(c)+(d)]/[(a)+(b)] erfüllt sein müssen.

Alle vorgenannten Mengenbereiche in Gew.-% sind hierbei auf die Gesamtmenge der im Mittel (A) enthaltenen niedrig ethoxylierten Fettalkohole (b) bezogen, die zum Gesamtgewicht des Mittels (A) in Relation gesetzt wird.

In einer weiteren ganz besonders bevorzugten Ausführungsform ist ein erfindungsgemäßes Mittel (A) somit dadurch gekennzeichnet, dass es - bezogen auf das Gesamtgewicht des Mittels (A) - (b) einen oder mehrere niedrig ethoxylierte C₁₆-C₁₈-Fettalkohole mit 1 bis 5 Ethoxy- Gruppen in einer Gesamtmenge von 0,05 bis 1,5 Gew.-%, bevorzugt von 0,05 bis 1,25 Gew.-%, weiter bevorzugt von 0,1 bis 1,0 Gew.-% und ganz besonders bevorzugt von 0,1 bis 0,75 Gew.-% enthält.

### Gewichtsverhältnis (a)/(b)

Betreffend die in den erfindungsgemäßen Mitteln (A) eingesetzten Gesamtmengen an hoch ethoxylierten Fettalkoholen (a) und niedrig ethoxylierten Fettalkoholen (b) müssen zwei Verhältnis- bedingungen erfüllt werden.

Die erste Verhältnisbedingung besteht darin, dass das Gewichtsverhältnis aus allen im Mittel (A) enthaltenen hoch ethoxylierten C₁₆-C₁₈-Fettalkoholen (a) zu allen im Mittel (A) enthaltenen niedrig ethoxylierten C₁₆-C₁₈-Fettalkoholen (b), d.h. das Gewichtsverhältnis (a)/(b), bei einem Wert von 10 bis 20 liegt.

Mit anderen Worten müssen die hoch ethoxylierten Fettalkohole (a) im Vergleich zu den niedrig ethoxylierten Fettalkoholen (b) in einem 10-fachen bis 20-fachen Gewichtsüberschuss im Mittel (A) eingesetzt werden.

Ohne auf diese Theorie festgelegt zu sein, wird vermutet, dass sich bedingt durch die Wahl dieser bestimmten Mengenverhältnisse an (a) und (b) (zusammen mit den weiteren Bestandteilen (c) und (d)) ein Emulsionssystem ausbildet, in dem die verschiedenen Emulgatoren (a) bis (d) in besonders stabilen Filmen oder Phasen vorliegen. Die auf diese Weise entstehende Emulsion besitzt eine besonders hohe Toleranz gegenüber variierenden Salzkonzentrationen. Dies hat zur Folge, dass der Einsatz verschiedener, in Salzform eingesetzter Mengen an Oxidationsfarbstoffen auf die rheologischen Eigenschaften der Emulsion besonders geringe Auswirkungen hat.

Als ganz besonders bevorzugt hat es sich in diesem Zusammenhang erwiesen, wenn das Gewichtsverhältnis aus allen im Mittel (A) enthaltenen hoch ethoxylierten C₁₆-C₁₈-Fettalkoholen (a) zu allen im Mittel (A) enthaltenen niedrig ethoxylierten C₁₆-C₁₈-Fettalkoholen (b), d.h. das Gewichtsverhältnis (a)/(b), bei einem Wert von 11 bis 19, bevorzugt von 12 bis 18, weiter bevorzugt von 13 bis 17 und ganz besonders bevorzugt von 14 bis 16 liegt.

In einer weiteren ganz besonders bevorzugten Ausführungsform ist ein erfindungsgemäßes Mittel (A) somit dadurch gekennzeichnet, dass das Gewichtsverhältnis aus allen im Mittel (A) enthaltenen hoch ethoxylierten C₁₆-C₁₈-Fettalkoholen (a) zu allen im Mittel (A) enthaltenen niedrig ethoxylierten C₁₆-C₁₈- Fettalkoholen (b), d.h. das Gewichtsverhältnis (a)/(b), bei einem Wert von 11 bis 19, bevorzugt von 12 bis 18, weiter bevorzugt von 13 bis 17 und ganz besonders bevorzugt von 14 bis 16 liegt.

Beispiel: eine Farbcreme enthält neben Ammoniak (e) und Oxidationsfarbstoffen (f)
(a) 3,0 Gew.-% Ceteth-50 und 1,0 Gew.-% Steareth-50 (Gesamtmenge an (a) = 4,0 Gew.-%)
(b) 0,2 Gew.-% Ceteth-2 und 0,2 Gew.-% Steareth-3 (Gesamtmenge an (b) = 0,4 Gew.-%) Das Gewichtsverhältnis (a)/(b) liegt bei 4,0 Gew.-%/ 0,4 Gew.-% = 10

Beispiel: eine Farbcreme enthält neben Ethanolamin (e) und Oxidationsfarbstoffen (f)
(a) 3,0 Gew.-% Ceteth-50 und 1,0 Gew.-% Steareth-50 (Gesamtmenge an (a) = 4,0 Gew.-%)
(b) 0,1 Gew.-% Ceteth-2 und 0,1 Gew.-% Steareth-3 (Gesamtmenge an (b) = 0,2 Gew.-%)
Das Gewichtsverhältnis (a)/(b) liegt bei 4,0 Gew.-%/ 0,2 Gew.-% = 20

### (c) Monoester aus Glycerin und C₁₄-C₂₀-Fettsäure

Als Bestandteil (c) enthalten die erfindungsgemäßen Mittel (A) einen oder mehrere Mono-ester aus Glycerin und einer C₁₄-C₂₀-Fettsäure.

Entsprechende Ester der Gruppe (c) werden somit jeweils durch Veresterung von einem Äquivalent (= Moläquivalent) Glycerin und einem Äquivalent (= Moläquivalent) C₁₄-C₂₀-Fettsäure hergestellt.

Unter C₁₄-C₂₀-Fettsäuren sind erfindungsgemäß gesättigte oder ungesättigte, unverzweigte oder verzweigte, C₁₄-C₂₀)-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Es zeichnen sich die Fettsäuremonoglyceride durch besondere Eignung aus, bei welchen eine Estergruppe ausgehend von Glycerin mit einer Fettsäure ausgebildet wird, die ausgewählt wird aus Tetradecansäure (Myristinsäure), Hexadecansäure (Palmitinsäure), Tetracosansäure (Lignocerinsäure), Octadecansäure (Stearinsäure), Eicosansäure (Arachinsäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Linolsäure [(9Z, 12Z)-Octadeca-9,12-diensäure,

Linolensäure [(9Z,12Z,15Z)-Octadeca-9,12,15-triensäure, Elaeostearinäure [(9Z,11E,13E)-Octadeca-9,11,3-triensäure] und/oder Arachidonsäure [(5Z,8Z,11Z,14Z)-Icosa-5,8,11,14-tetraensäure].

Ganz besonders bevorzugt enthält das erfindungsgemäße Mittel (A) (c) einen oder mehrere Monoester aus Glycerin und einer C₁₄-C₂₀-Fettsäure der Formel (III), 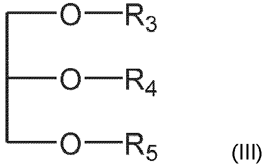 worin
R3, R4, R5 unabhängig voneinander für ein Wasserstoffatom oder eine Acylgruppe - C(O)R₆ stehen, in der R₆ für eine unverzweigte oder verzweigte, gesättigte oder ungesättigte C₁₃-C₁₉-Alkylgruppe, bevorzugt eine unverzweigte, gesättigte C₁₅-C₁₇-Alkylgruppe, steht, mit der Maßgabe, dass zwei der Reste aus R3, R4 und R5 für ein Wasserstoffatom stehen und der dritte Rest für eine Acylgruppe -C(O)R₆ steht.

In einer weiteren ganz besonders bevorzugten Ausführungsform ist ein erfindungsgemäßes Mittel (A) somit dadurch gekennzeichnet, dass es (c) einen oder mehrere Monoester aus Glycerin und einer C₁₄-C₂₀-Fettsäure der Formel (III) enthält,
worin 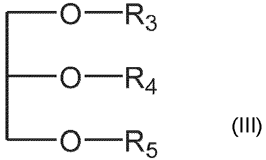 R3, R4, R5 unabhängig voneinander für ein Wasserstoffatom oder eine Acylgruppe - C(O)R₆ stehen, in der R₆ für eine unverzweigte oder verzweigte, gesättigte oder ungesättigte C₁₃-C₁₉-Alkylgruppe, bevorzugt eine unverzweigte, gesättigte C₁₅-C₁₇-Alkylgruppe, steht, mit der Maßgabe, dass zwei der Reste aus R3, R4 und R5 für ein Wasserstoffatom stehen und der dritte Rest für eine Acylgruppe -C(O)R₆ steht.

Als besonders gut geeignete Verbindung der Formel (III) kann beispielsweise einer der Monoester aus Glycerin und einer C₁₄-C₂₀-Fettsäure eingesetzt werden, der aus der Gruppe der Formeln (lila) bis (IIId) ausgewählt wird.

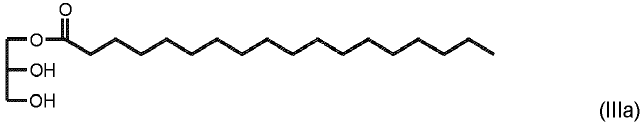

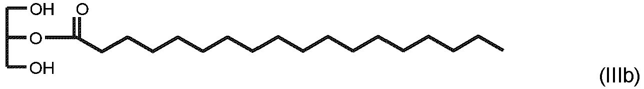

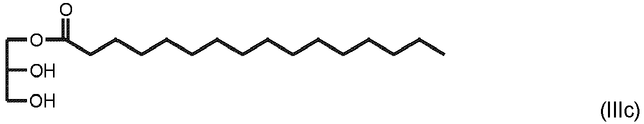

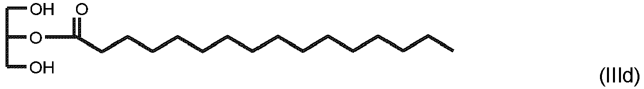

Die Verbindungen der Formeln (lila) bis (IIId) sind auch unter den Namen Glycerylmonostearat und Glycerylmonopalmitat bekannt.

Die Monoester aus Glycerin und einer C₁₄-C₂₀-Fettsäure (c) können zusammen mit den Inhaltsstoffen (a), (b) und (d) das erfindungsgemäße Emulgartorsystem ausbilden, welches eine stabile Einarbeitung der weiteren Bestandteile (e) und (f) (Alkalisierungsmittel und Oxidationsfarbstoffe) gewährleistet. Bevorzugt werden daher auch die Fettsäure-Monoglyceride (c) in bestimmten Mengenbereichen im erfindungsgemäßen Mittel (A) eingesetzt.

Besonders stabile und salztolerante Emulsionen konnten erhalten werden, wenn die Mittel (A) einen oder mehrere Monoester aus Glycerin und einer C₁₄-C₂₀-Fettsäure (c) in einer Gesamtmenge von 0,1 bis 5,0 Gew.-%, bevorzugt von 0,1 bis 2,5 Gew.-%, weiter bevorzugt von 0,1 bis 1,3 Gew.-% und ganz besonders bevorzugt von 0,2 bis 0,7 Gew.-% enthielten.

Die Mengenangaben in Gew.-% beziehen sich hierbei wieder auf die Gesamtmenge aller im Mittel (A) enthaltenen Fettsäure-Monoglyceride (c), die zum Gesamtgewicht des Mittels (A) in Relation gesetzt wird. Auch bei diesen Mengenangaben betreffend die im Mittel enthaltene Gesamtmenge an Monoester aus Glycerin und einer C₁₄-C₂₀-Fettsäure (c) gilt wieder die Voraussetzung, dass zusätzlich die Verhältnisbedingung [(c)+(d)]/[(a)+(b)] erfüllt sein muss.

In einer weiteren ganz besonders bevorzugten Ausführungsform ist ein erfindungsgemäßes Mittel (A) dadurch gekennzeichnet, dass es - bezogen auf das Gesamtgewicht des Mittels - (c) einen oder mehrere Monoester aus Glycerin und einer C₁₄-C₂₀-Fettsäure in einer Gesamtmenge von 0,1 bis 5,0 Gew.-%, bevorzugt von 0,1 bis 2,5 Gew.-%, weiter bevorzugt von 0,1 bis 1,3 Gew.-% und ganz besonders bevorzugt von 0,2 bis 0,7 Gew.-% enthält.

### (d) C₁₆-C₁₈-Fettalkohole

Für die Ausbildung der Emulsion wesentlich ist weiterhin die Anwesenheit mindestens eines C₁₆-C₁₈-Fettalkohols (d). Deshalb enthalten die erfindungsgemäßen Mittel (A) als weiteren wesentlichen Inhaltsstoff (d) einen oder mehrere C₁₆-C₁₈-Fettalkohole. Bei den C₁₆-C₁₈-Fettalkoholen handelt es sich um gesättigte, ein- oder mehrfach ungesättigte, lineare oder verzweigte Alkanole mit 16 bis 18 C-Atomen.

Beispiele für bevorzugte lineare, gesättigte C₁₆- C₁₈-Fettalkohole sind Hexadecan-1-ol (Hexadecylalkohol, Cetylalkohol, Palmitylalkohol), Octadecan-1-ol (Octadecylalkohol, Stearylalkohol) und deren Gemische.

Bevorzugte lineare, ungesättigte Fettalkohole sind (9*Z*)-Octadec-9-en-1-ol (Oleylalkohol), (9E)-Octadec-9-en-1-ol (Elaidylalkohol), (9*Z*,12*Z*)-Octadeca-9,12-dien-1-ol (Linoleylalkohol) und (9*Z*,12*Z*,15*Z*)-Octadeca-9,12,15-trien-1-ol (Linolenoylalkohol).

In einer besonders bevorzugten Ausführungsform enthält das erfindungsgemäße Mittel (A) mindestens einen linearen, gesättigten C₁₆-C₁₈-Fettalkohol.

Die C₁₆-C₁₈-Fettalkohole (d) stellen in den erfindungsgemäßen Farbcremes den lipophilsten Bestandteil des aus den Komponenten (a) bis (d) bestehenden Emulgator-Systems dar. Zur Optimierung dieses Emulgator-Systems werden auch der oder die C₁₆-C₁₈-Fettalkohole (d) besonders bevorzugt in bestimmten Mengenbereichen im erfindungsgemäßen Mittel (A) eingesetzt. Auch bei diesen Mengenangaben betreffend die im Mittel enthaltene Gesamtmenge an C₁₆-C₁₈-Fettatkoholen (d) gilt wieder die Voraussetzung, dass zusätzlich die Verhältnisbedingung [(c)+(d)]/[(a)+(b)] erfüllt sein muss.

Emulsionen mit ganz besonders guter Lagerstabilität konnten erhalten werden, wenn die Mittel (A) - bezogen auf ihr Gesamtgewicht- (d) einen oder mehrere C₁₆-C₁₈-Fettalkohole in einer Gesamtmenge von 0,1 bis 20,0 Gew.-%, bevorzugt von 5,0 bis 15,0 Gew.-%, weiter bevorzugt von 10,0 bis 13,0 Gew.-% und ganz besonders bevorzugt von 10,5 bis 12,5 Gew.-% enthielten. Hierbei beziehen sich alle Angaben in Gew.-% wieder auf die Gesamtmenge der im Mittel (A) eingesetzten C₁₆-C₁₈-Fettalkohole (d), die zum Gesamtgewicht des Mittels in Relation gesetzt wird.

In einer weiteren ganz besonders bevorzugten Ausführungsform ist ein erfindungsgemäßes Mittel (A) somit dadurch gekennzeichnet, dass es - bezogen auf das Gesamtgewicht des Mittels (A) - (d) einen oder mehrere C₁₆-C₁₈-Fettalkohole in einer Gesamtmenge von 0,1 bis 20,0 Gew.-%, bevorzugt von 5,0 bis 15,0 Gew.-5, weiter bevorzugt von 10,0 bis 13,0 Gew.-% und ganz besonders bevorzugt von 10,5 bis 12,5 Gew.-% enthält.

### Gewichtsverhältnis [(c)+(d)]/[(a)+(b)]

Betreffend die Einsatzmengen der Emulgatorbestandteile (a), (b), (c) und (d) muss als zweite Verhältnisbedingung erfüllt sein, dass das Gewichtsverhältnis aus der Summe aller im Mittel (A) enthaltenen Bestandteile [(c)+(d)] zur Summe aller im Mittel enthaltenden ethoxylierten Fettalkohole [(a)+(b)], d.h. das Gewichtsverhältnis [(c)+(d)]/[(a)+(b)], bei einem Wert von 2 bis 5 liegt.

Diese Verhältnisbedingung besagt, dass die Summe aus Fettsäure-Monoglyceriden (c) und C₁₆-C₁₈-Fettalkoholen (d) im Vergleich zur Summe der hoch und niedrig ethoxylierten Fettalkohole (a) und (b) um den Faktor 2 bis 5 höher ist. Wenn diese Verhältnis-Bedingung eingehalten wird, bildet sich eine O/W-Emulsion mit einer Abmischung aus nichtionischen lipophilen und hydrophilen Emulgator-Bestandteilen (a) bis (d) aus, die Variationen des Salz-Gehaltes ganz besonders gut toleriert. Entsprechende Emulsionen bleiben bei niedrigen Salzgehalten, insbesondere aber auch bei hohen Salzgehalten besonders stabil. Zudem ändert sich die Viskosität einer Farbcreme, die einen hohen Salzgehalt besitzt, im Vergleich zu einer ansonsten gleichen Farbcreme, die aber einen niedrigeren Salzgehalt besitzt, nur unwesentlich. Die Stabilität der Viskosität unabhängig vom Salzgehalt der Emulsion stellt in der Produktion eines viele verschiedene Nuancen umfassenden Färbe-Portfolios einen wesentlichen Vorteil dar.

Als explizit ganz besonders bevorzugt hat sich herausgestellt, wenn das Gewichtsverhältnis aus der Summe aller im Mittel (A) enthaltenen Bestandteile [(c)+(d)] zur Summe aller im Mittel (A) enthaltenen ethoxylierten Fettalkohole [(a)+(b)], d.h. das Gewichtsverhältnis [(c)+(d)]/[(a)+(b)] bei einem Wert von 2,0 bis 4,5, bevorzugt von 2,0 bis 4,0, weiter bevorzugt von 2,0 bis 3,5 und ganz besonders bevorzugt von 2,5 bis 3,0 liegt.

In einer weiteren ganz besonders bevorzugten Ausführungsform ist ein erfindungsgemäßes Mittel (A) somit dadurch gekennzeichnet, dass das Gewichtsverhältnis aus der Summe aller im Mittel (A) enthaltenen Bestandteile [(c)+(d)] zur Summe aller im Mittel (A) enthaltenen ethoxylierten Fettalkohole [(a)+(b)], d.h. das Gewichtsverhältnis [(c)+(d)]/[(a)+(b)] bei einem Wert von 2,0 bis 4,5, bevorzugt von 2,0 bis 4,0, weiter bevorzugt von 2,0 bis 3,5 und ganz besonders bevorzugt von 2,5 bis 3,0 liegt.

Beispiel: eine Farbcreme enthält neben Ammoniak (e) und Oxidationsfarbstoffen (f)
(a) 4,0 Gew.-% Ceteareth-50
(b) 0,4 Gew.-% Ceteareth-2
(c) 0,5 Gew.-% Glycerylmonostearat
(d) 11,0 Gew.-% Cetearylalkohol

Das Gewichtsverhältnis [(c)+(d)]/[(a)+(b)] liegt bei 11,5 / 4,4 = 2,6

Beispiel: eine Farbcreme enthält neben Ethanolamin (e) und Oxidationsfarbstoffen (f)
(a) 4,5 Gew.-% Ceteth-50
(b) 0,2 Gew.-% Ceteth-2
(c) 0,7 Gew.-% Glycerylmonostearat
(d) 12,0 Gew.-% Cetearylalkohol

Das Gewichtsverhältnis [(c)+(d)]/[(a)+(b)] liegt bei 12,7 / 4,7 = 2,7

### (e) Alkalisierungsmittel

Die erfindungsgemäßen Mittel (A) werden als Farbcremes bei der oxidativen Färbung von Keratinfasern, insbesondere Haaren, eingesetzt. Die Oxidationsfärbung von Haaren findet in der Regel bei neutralem, insbesondere bei alkalischem pH-Wert statt. Aus diesem Grund sind die erfindungsgemäßen Mittel alkalisch eingesetllt und enthalten mindestens ein Alkalisierungsmittel (e).

Die erfindungsgemäßen Farbcremes weisen bevorzugt einen pH-Wert im Bereich von 7 bis 12, bevorzugt einen pH-Wert im Bereich von 8,0 bis 11,5 auf. Bei den pH-Werten im Sinne der vorliegenden Erfindung handelt es sich um pH-Werte, die bei einer Temperatur von 22°C gemessen wurden.

Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden ausgewählt aus 2-Aminoethan-1-ol (Monoethanolamin), 2-Amino-2-methylpropan-1-ol, 2-Amino-2-methyl-propan-1,3-diol und Triethanolamin. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat, bevorzugt Natriumhydroxid und/oder Kaliumhydroxid. Die basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin und D/L-Arginin. Schließlich ist ein weiteres bevorzugtes Alkalisierungsmittel Ammoniak.

In einer weiteren ganz besonders bevorzugten Ausführungsform ist ein erfindungsgemäßes Mittel (A) somit dadurch gekennzeichnet, dass es ein oder mehrere Alkalisierungsmittel (e) aus der Gruppe aus Ammoniak, 2-Aminoethan-1-ol, 2-Amino-2-methylpropan-1-ol, 2-Amino-2-methyl-propan-1,3- diol , L-Arginin, D-Arginin, D/L-Arginin, L-Lysin, D-Lysin und D/L-Lysin enthält.

### Oxidationsfarbstoffvorprodukte

Bei den erfindungsgemäßen Mitteln (A) handelt es sich um Farbcremes zur oxidativen Färbung von keratinischen Fasern, insbesondere menschlichen Haaren. Die oxidative Färbung wird durch Einsatz mindestens eines Oxidationsfarbstoffvorproduktes (f) erzielt. Als weiteren wesentlichen Bestandteil (f) enthalten die erfindungsgemäßen Mittel (A) daher ein oder mehrere Oxidationsfarbstoff-vorprodukte. Oxidationsfarbstoffvorprodukte können in Entwickler und Kuppler unterteilt werden, wobei die Entwickler aufgrund ihrer größeren Empfindlichkeit gegenüber Sauerstoff meist in Form ihrer physiologisch verträglichen farbverändernde Salze (z.B. in Form ihrer Sulfate, Hydrogen- sulfate, Chloride oder Bromide) eingesetzt werden.

Kupplerkomponenten bilden im Rahmen der oxidativen Färbung allein keine signifikante Färbung aus, sondern benötigen stets die Gegenwart von Entwicklerkomponenten. Da Kuppler gegenüber Sauerstoff nicht so empfindlich sind wie Entwickler, können sie zwar ebenfalls in Form ihrer Farbverändernde Salze in den Zubereitungen eingesetzt werden, werden oftmals aber auch in freier Form (d.h. nicht in Salzform) eingesetzt.

Im Zuge der zu dieser Erfindung führenden Arbeiten hat sich herausgestellt, dass insbesondere Nuancen mit hohem Mengenanteil an Oxidationsfarbstoffvorprdodukten, d.h. Braun-Nuancen, Schwarz-Nuancen oder andere dunkle Nuancen, sehr gut durch die erfindungsgemäßen Mittel stabilisiert werden können. Dieser Effekt ist besonders dann ausgeprägt, wenn Oxidationsfarbstoffvorprodukte in Form ihrer physiologisch verträglichen Salze eingesetzt werden.

Bei Oxidationsfarbstoffvorprodukten vom Entwicklertyp handelt es sich üblicherweise um Derivate des p-Phenylendiamins, das p-Aminophenols oder um heterozyklische Verbindungen mit mindestens einer, bevorzugt mindestens zwei Aminogruppen. Zur Überführung in ihre Salze werden die in diesen Strukturen enthaltenen Aminogruppen protoniert und besitzen zur Neutralisation dieser positiven Ladung die entsprechenden Äquivalente an Sulfat-Anionen, Hydrogensulfat-Anionen, Chlorid-Anionen und/oder Bromid-Anionen.

Bei p-Toluylendiamin Sulfat handelt es sich beipsielsweise um die Verbindung Toluylendiamin x H₂SO₄. Beide Aminogruppen liegen protoniert (in Form von Ammoniumionen) vor und die nun im Molekül enthaltenen beiden kationischen Ladungen werden durch ein Sulfat-Anion (SO₄²⁻) neutralisiert. Bei p-Toluylendiamin Monohydrochlorid handelt es sich demnach um die Verbindung Toluylendiamin x HCl. Eine der beiden Aminogruppen liegt protoniert vor und besitzt ein Chlorid als Gegenion. Bei p-Toluylendiamin Dihydrochlorid handelt es sich um die Verbindung Toluylendiamin x 2 HCl. Beide Aminogruppen liegt protoniert vor und besitzten zwei Chloride als Gegenion. Die Salze der weiteren Oxidationsfarbstoffe vom Entwicklertyp setzen sich in analoger Weise zusammen.

Bevorzugte physiologisch verträgliche farbverändernde Salze von Entwicklern sind beispielsweise Phenylendiamin Sulfat, Phenylendiamin Monohydrochlorid, Phenylendiamin Dihydrochlorid, p-Toluylendiamin Sulfat, p-Toluylendiamin Monohydrochlorid, p-Toluylendiamin Dihydrochlorid, 2-(2-Hydroxyethyl)-p-phenylendiamin Sulfat, 2-(2-Hydroxyethyl)-p-phenylendiamin Monohydrochlorid, 2-(2-Hydroxyethyl)-p-phenylendiamin Dihydrochlorid, N,N-Bis-(2-hydroxyethyl)-p-phenylendiamin Sulfat, N,N-Bis-(2-hydroxyethyl)-p-phenylendiamin Monohydrochlorid, N,N-Bis-(2-hydroxyethyl)-p-phenylendiamin Dihydrochlorid, 2-Methoxymethyl-p-phenylendiamin Sulfat, 2-Methoxymethyl-p-phenylendiamin Monohydrochlorid, 2-Methoxymethyl-p-phenylendiamin Dihydrochlorid, p-Aminophenol Hydrogensulfat, p-Aminophenol Monohydrochlorid, 4-Amino-3-methylphenol

Hydrogensulfat, 4-Amino-3-methylphenol Chlorid, 2,4,5,6-Tetraaminopyrimidin Monosulfat, 2,4,5,6-Tetraaminopyrimidin Disulfat, 2,4,5,6-Tetraaminopyrimidin Monohydrochlorid, 2,4,5,6-Tetraaminopyrimidin Dihydrochlorid, 2,4,5,6-Tetraaminopyrimidin Trihydrochlorid, 2,4,5,6-Tetraaminopyrimidin Tetrahydrochlorid, 4-Hydroxy-2,5,6-triaminopyrimidin Sulfat, 4-Hydroxy-2,5,6-triamino-pyrimidin Monohydrochlorid, 4-Hydroxy-2,5,6-triaminopyrimidin Diydrochlorid, 4-Hydroxy-2,5,6-triaminopyrimidin Trihydrochlorid, 4,5-Diamino-1-(2-hydroxyethyl)-1H-pyrazol Sulfat, 4,5-Diamino-1-(2-hydroxyethyl)-1H-pyrazol Monohydrochlorid und/oder 4,5-Diamino-1-(2-hydroxyethyl)-1H-pyrazol Dihydrochlorid.

In einer weiteren ganz besonders bevorzugten Ausführungsform ist ein erfindungsgemäßes Mittel (A) dadurch gekennzeichnet, dass es ein oder mehrere Oxidationsfarbstoffvorprodukte (f) aus der Gruppe aus Phenylendiamin Sulfat, Phenylendiamin Monohydrochlorid, Phenylendiamin Dihydrochlorid, p-Toluylendiamin Sulfat, p-Toluylendiamin Monohydrochlorid, p-Toluylendiamin Dihydrochlorid, 2-(2-Hydroxyethyl)-p-phenylendiamin Sulfat, 2-(2-Hydroxyethyl)-p-phenylendiamin Monohydrochlorid, 2-(2-Hydroxyethyl)-p-phenylendiamin Dihydrochlorid, N,N-Bis-(2-hydroxyethyl)- p-phenylendiamin Sulfat, N,N-Bis-(2-hydroxyethyl)-p-phenylendiamin Monohydrochlorid, N,N-Bis- (2-hydroxyethyl)-p-phenylendiamin Dihydrochlorid, 2-Methoxymethyl-p-phenylendiamin Sulfat, 2- Methoxymethyl-p-phenylendiamin Monohydrochlorid, 2-Methoxymethyl-p-phenylendiamin Dihydrochlorid, p-Aminophenol Hydrogensulfat, p-Aminophenol Monohydrochlorid, 4-Amino-3- methylphenol Hydrogensulfat, 4-Amino-3-methylphenol Chlorid, 2,4,5,6-Tetraaminopyrimidin Monosulfat, 2,4,5,6-Tetraaminopyrimidin Disulfat, 2,4,5,6-Tetraaminopyrimidin Monohydrochlorid, 2,4,5,6-Tetraaminopyrimidin Dihydrochlorid, 2,4,5,6-Tetraaminopyrimidin Trihydrochlorid, 2,4,5,6-Tetraaminopyrimidin Tetrahydrochlorid, 4-Hydroxy-2,5,6-triaminopyrimidin Sulfat, 4-Hydroxy-2,5,6-triaminopyrimidin Monohydrochlorid, 4-Hydroxy-2,5,6-triaminopyrimidin Diydrochlorid, 4-Hydroxy-2,5,6-triaminopyrimidin Trihydrochlorid, 4,5-Diamino-1-(2-hydroxyethyl)-1H-pyrazol Sulfat, 4,5-Diamino-1-(2-hydroxyethyl)-1H-pyrazol Monohydrochlorid und/oder 4,5-Diamino-1-(2-hydroxyethyl)-1H-pyrazol Dihydrochlorid enthält.

Abhängig vom gewünschten Farbresultat werden Oxidationsfarbstoffvorprodukte vom Entwickler- und vom Kupplertyp in unterschiedlich hohen Mengenanteilen im Färbemittel eingesetzt.

Wird die Färbung in einer Blondnuance gewünscht, so genügt meist der Einsatz Oxidationsfarbstoffvorprodukte in einer Gesamtmenge unterhalb von 0,3 Gew.-%.

Wünscht der Anwender jedoch die Färbung in einem sehr dunklen Farbton, beispielsweise in einer Dunkelbraun-Nuance oder in einer Schwarz-Nuance, so macht dies den Einsatz von Oxidationsfarbstoffvorprodukten in einer Gesamtmenge von mindestens 2,0 Gew.-%, oft 3,0 Gew.-%, und bei besonders dunklen Nuancen (schwarz) sogar oberhalb von 4,5 Gew.-% (bezogen auf das Gesamtgewicht des erfindungsgemäßen Mittel, d.h. der alkalisch eingestellten Farbcreme) erforderlich.

Je höher der Farbstoffgehalt ist, desto schwieriger ist es, das Mittel zu stabilisieren. In diesem Zusammenhang hat sich herausgestellt, dass vor allem die Stabilisierung von Braun- bzw. Schwarz-Nuancen durch das zuvor beschriebene, die Bestandteile (a) bis (d) enthaltende Emulgatorsystem sehr gut möglich ist. Bevorzugt enthalten die erfindungsgemäßen Mittel (A) daher es ein oder mehrere Oxidationsfarbstoffvorprodukte (f) aus der Gruppe der Sulfate, Chloride und Bromide in einer Gesamtmenge von 0,01 bis 5,5 Gew.-%, bevorzugt von 0,7 bis 5,0 Gew.-%, weiter bevorzugt von 0,9 bis 4,5 Gew.-% und ganz besonders bevorzugt von 2,1 bis 3,4 Gew.-%.

In einer weiteren ganz besonders bevorzugten Ausführungsform ist ein erfindungsgemäßes Mittel dadurch gekennzeichnet, dass es - bezogen auf das Gesamtgewicht des Mittels - es ein oder mehrere Oxidationsfarbstoffvorprodukte (f) aus der Gruppe der Sulfate, Chloride und Bromide in

einer Gesamtmenge von 0,01 bis 5,5 Gew.-%, bevorzugt von 0,7 bis 5,0 Gew.-%, weiter bevorzugt von 0,9 bis 4,5 Gew.-% und ganz besonders bevorzugt von 2,1 bis 3,4 Gew.-% enthält.

Oxidationsfarbstoffvorprodukte vom Entwicklertyp können als alleinige farbverändernde Verbindungen im erfindungsgemäßen Mittel enthalten sein. Es ist erfindungsgemäß jedoch bevorzugt, wenn das Färbemittel (A) zusätzlich mindestens ein Oxidationsfarbstoffvorprodukt vom Kupplertyp (kurz Kuppler genannt) enthält.

Kupplerkomponenten bilden im Rahmen der oxidativen Färbung allein keine signifikante Färbung aus, sondern benötigen stets die Gegenwart von Entwicklerkomponenten. Kupplerkomponenten im Sinne der Erfindung erlauben mindestens eine Substitution eines chemischen Restes des Kupplers durch die oxidierte Form der Entwicklerkomponente. Dabei bilden sich kovalente Bindungen zwischen Kuppler- und Entwicklerkomponente aus.

Als erfindungsgemäß geeignete Kupplerkomponente wird bevorzugt mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin.

Gemische aus zwei oder mehreren Verbindungen aus einer oder mehreren dieser Klassen sind im Rahmen dieser Ausführungsform ebenso erfindungsgemäß.

In einer weiteren Ausführungsform ist ein erfindungsgemäßes Mittel (A) gekennzeichnet, dass es mindestens ein Oxidationsfarbstoffvorprodukt vom Kuppler-Typ enthält, welches ausgewählt wird aus der Gruppe aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diamino-phenoxy)ethanol, 1,3-Bis(2,4-diaminophenoxy)propan, 1-Methoxy-2-amino-4-(2-hydroxyethyl-amino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)-amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin und deren physiologisch verträglichen Farbverändernde Salzen.

Zusätzlich zu den Oxidationsfarbstoffvorprodukten oder anstatt dieser können die erfindungsgemäßen Mittel (A) mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Direktziehende Farbstoffe können in anionische, kationische und nichtionische direktziehende Farbstoffe unterteilt werden.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2- nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Farbverändernde Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe tragen mindestens eine negative Ladung und werden in der Literatur auch als Säurefarbstoffe bezeichnet. Bevorzugte anionische direktziehende Farbstoffe sind die unter den internationalen Bezeichnungen bzw. Handelsnamen Bromphenolblau, Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Kationische Farbstoffe zeichnen sich durch das Vorhandensein von mindestens einer positiven Ladung aus. In der englischen Literatur werden kationische Farbstoffe auch "basic dyes" genannt. Bevorzugte kationische direktziehende Farbstoffe sind Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Yellow 87, Basic Orange 31 und Basic Red 51.

Wie bereits zuvor beschrieben wird die Stabilisierung von Emulsionen oft umso schwieriger, je höher der Salzgehalt einer Emulsion ist. Daher lassen sich Emulsionen, in denen hohe Mengen an kationischen direktziehenden Farbstoffen eingesetzt werden, üblicherweise auch schwieriger stabilisieren als Emulsionen mit niedrigem Farbstoffgehalt oder Emulsionen, die nur nichtionische direktziehende Farbstoffe enthalten.

Im Zuge der durchgeführten Arbeiten hat sich gezeigt, dass sich die in Emulsionsform vorliegenden Färbemittelinsbesondere auch dann sehr gut stabilisieren ließen, wenn das Mittel (A) zusätzlich einen kationischen und/oder anionischen direktziehenden Farbstoff enthielt.

Prinzipiell können die direktziehenden Farbstoffe in einer Gesamtmenge von 0,001 bis 10 Gew.-% im erfindungsgemäßen Mittel (A) enthalten sein.

### Viskosität

Wie zuvor beschrieben, kann durch den Einsatz der nichtionischen Emulgatoren (a) bis (d) in Farbcremes, welche Alkalisierungsmittel (e) und Oxidaitonsfarbstoffvorprodukte (f) enthalten, eine besonders stabile Emulsion erzeugt werden, die auch eine ausgezeichnete Viskositäts-Stabilität besitzt. Mit Viskositäts-Stabilität ist in diesem Zusammenhang gemeint, dass die Emulsion auf Änderungen des Salzgehaltes nur mit minimalen Viskositäts-Schwankungen reagiert.

Die Viskositäten wurden bei den zu dieser Erfindung führenden Arbeiten mit einem Viskosimeter des Typs Haake Rheostress 6000 bei 20 °C gemessen (20 °C / Haake Rheostress 6000 / gemessen mit Cone 35/1 Geometrie, Durchmesser 35 mm und 1° Winkel / Scherrate 7,2 s⁻¹).

Durch Einsatz der Bestandteile (a) bis (d) in den zuvor beschriebenen Mengenbereichen und - verhältnissen konnte sichergestellt werden, dass die Viskositäten der Farbremes im Bereich von 10.000 bis 30.000 mPas, bevorzugt von 12.000 bis 25.000 mPas und ganz besonders bevorzugt von 16.000 bis 21.000 mPas lagen (20 °C / Haake Rheostress 6000 / gemessen mit Cone 35/1 Geometrie, Durchmesser 35 mm und 1° Winkel / Scherrate 7,2 s⁻¹).

In einer weiteren ganz besonders bevorzugten Ausführungsform ist ein erfindungsgemäßes Mittel (A) dadurch gekennzeichnet, dass es eine Viskosität von 10.000 bis 30.000 mPas, bevorzugt von 12.000 bis 25.000 mPas und ganz besonders bevorzugt von 16.000 bis 21.000 mPas besitzt (20 °C / Haake Rheostress 6000 / gemessen mit Cone 35/1 Geometrie, Durchmesser 35 mm und 1° Winkel / Scherrate 7,2 s⁻¹).

### Verzweigte Fettalkohole

Im Rahmen einer weiteren Ausführungsform kann es sich als vorteilhaft erweisen, wenn das erfindungsgemäße Mittel (A) zusätzlich den verzweigten Fettalkohole 2-Octyldodecanol (d.h. einen verzweigten C₂₀-Fettalkohol) enthält.

Die Menge des im Mittel (A)eingesetzten 2-Octyldodecanols liegt hierbei - bezogen auf das Gesamtgewicht des Mittels - bevorzugt in einem Bereich von 0,1 bis 10 Gew.-%, bevorzugt von 0,5 bis 5,0 Gew.-% und ganz besonders bevorzugt von 1,0 bis 3,0 Gew.-%.

In einer weiteren ganz besonders bevorzugten Ausführungsform ist ein erfindungsgemäßes Mittel (A) dadurch gekennzeichnet, dass es - bezogen auf das Gesamtgewicht des Mittels - 0,1 bis 10 Gew.-%, bevorzugt von 0,5 bis 5,0 Gew.-% und ganz besonders bevorzugt von 1,0 bis 3,0 Gew.-% Octyldodecanol enthält.

### Mehrkomponenten-Verpackunqseinheit (Kit-of-Parts)

Bei den erfindungsgemäßen Mitteln handelt es sich um Mittel zur oxidativen Farbveränderung von keratinischen Fasern. Insbesondere handelt es sich um Mittel zur Färbung, vor allem zu oxidativen Färbung, von menschlichen Haaren. Zur Initiierung des oxidativen Färbeprozesses und zur Ausbildung der Oxidationsfarbstoffe wird die Farbcreme kurz vor der Anwendung mit einer Oxidationsmittelzubereitung vermischt. Auf diese Weise wird das anwendungsbereite oxidative Färbemittel hergestellt, welches auf die Haare des Anwenders aufgetragen wird.

Zur Vermeidung von Inkompatibilitäten und zur Verhinderung einer vorzeitigen, unerwünschten Farbstoffbildung werden die Farbcreme (als Mittel (A) bezeichnet) und die für die oxidative Färbung notwendige Oxidationsmittelzubereitung (Mittel (B)) stets getrennt voneinander konfektioniert und erst kurz vor der Anwendung mit einander in Kontakt gebracht. Für den Verbraucher werden die beiden Komponenten in Form einer Mehrkomponenten- Verpackungseinheit (Kit-of-parts) bereitgestellt.

### Oxidationsmittelzubereitung (B)

Die Oxidationsmittelzubereitung (B) enthält als Oxidationsmittel Wasserstoffperoxid. Das Wasserstoffperoxid kann entweder als Wasserstoffperoxid selbst oder auch in Form seiner festen Anlagerungsprodukte an organische oder anorganische Verbindungen, wie Harnstoff, Melamin sowie Natriumborat.

Bevorzugt beträgt die Menge an Oxidationsmittel in der Oxidationsmittelzubereitung (B) - bezogen auf das Gesamtgewicht der Oxidationsmittelzubereitung (B) - 0,5 bis 12 Gew.-%, bevorzugt 2 bis 10 Gew.-% insbesondere bevorzugt 3 bis 6 Gew.-% (berechnet als 100 %-iges H₂O₂).

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 85 Gew.-%, weiter bevorzugt 60 bis 85 Gew.-% und insbesondere 70 bis 85 Gew.-% Wasser enthält.

Weiterhin hat es sich als vorteilhaft erweisen, wenn die Oxidationsmittelzubereitung (B) mindestens einen Stabilisator oder Komplexbildner enthalten. Gebräuchliche und im Rahmen der vorliegenden Erfindung bevorzugte Komplexbildner und Stabilisatoren sind beispielsweise Polyoxycarbonsäuren, Polyamine, Ethylendiamintetraessigsäure (EDTA), N-Hydroxyethylethylendiamintriessigsäure, Diethylentriaminpentaessigsäure (DTPA), Ethylendiamindibernsteinsäure (EDDS), Hydroxyethyliminodiessigsäure, Nitridodiessigsäure-3-propionsäure, Isoserindiessigsäure, N,N-Di-(2-hydroxyethyl)glycin, N-(1,2-Dicarboxy-2-hydroxyethyl)glycin, N-(1,2-Dicarboxy-2-hydroxyethyl)asparaginsäure oder Nitrilotriessigsäure (NTA), Ethylendiamindiglutarsäure (EDGA), 2-Hydroxypropylendiamindibernsteinsäure (HPDS), Glycinamid-N,N'-dibernsteinsäure (GADS), Ethylendiamin-N-N'-diglutarsäure (EDDG), 2-Hydroxypropylendiamin-N-N'-dibernsteinsäure (HPDDS), Diaminoalkyldi-(sulfobernsteinsäure) (DDS), Ethylendicysteinsäure (EDC), Ethylendiamin-N-N'-bis(ortho-hydroxyphenyl)essigsäure (EDDHA), N-2-Hydroxyethylamin- N,N-diessigsäure, Glyceryliminodiessigsäure, Iminodiessigsäure-N-2-hydroxypropylsulfonsäure, Asparaginsäure-N-carboxymethyl-N-2,5-hydroxypropyl-3-sulfonsäure,β-Alanin-N,N'-diessigsäure, Asparaginsäure-N,N'-diessigsäure, Asparaginsäure-N-monoessigsäure, Dipicolinsäure, sowie deren Farbverändernde Salze und/oder Derivate, geminale Diphosphonsäuren wie 1- Hydroxyethan-1,1-diphosphonsäure (HEDP), deren höhere Homologe mit bis zu 8 Kohlenstoff- atomen sowie Hydroxy- oder Aminogruppen-haltige Derivate hiervon und 1-Aminoethan-1,1- diphosphonsäure, deren höhere Homologe mit bis zu 8 Kohlenstoffatomen sowie Hydroxy- oder Aminogruppen-haltige Derivate, Aminophosphonsäuren wie Ethylendiamintetra(methylen- phosphonsäure) (EDTMP), Diethylen-triaminpenta(methylenphosphonsäure) (DTPMP) sowie deren höhere Homologe, oder Nitrilotri(methylenphosphonsäure), Phosphonopolycarbonsäuren wie 2-Phosphonobutan-1,2,4-tricarbonsäure, Cyclodextrine, sowie Alkalistannate (Natriumstannat), Alkalipyrophosphate (Tetranatriumpyrophosphat, Dinatriumpyrophosphat), Alkaliphosphate (Natriumphosphat), und Phosphorsäure sowie deren Farbverändernde Salze.

### Weitere Inhaltsstoffe

Die erfindungsgemäßen Mittel (A) und/oder die Oxidationsmittelzubereitung (B) können zusätzlich weitere Wirk-, Hilfs- und Zusatzstoffe enthalten, die von den zuvor beschriebenen Bestandteilen (a) bis (f) verschieden sind. Dies können beispeilsweise sein: kationische Tenside, amphotere Tenside, anionische Tenside, nichtionische Tenside (die von den Bestandteilen (a) bis (d) verschieden sind), anionische, nichtionische und/oder kationische Polymere, Strukturanten wie Glucose, Maleinsäure und Milchsäure, Parfümöle, faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Farbverändernde Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Vitamine, Provitamine und Vitaminvorstufen; Pflanzenextrakte; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und - distearat sowie PEG-3-distearat; Pigmente sowie Treibmittel wie Propan-Butan-Gemische, N₂O, Dimethylether, CO₂ und Luft.

Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht des Mittels (A) bzw. der Oxidationsmittelzubereitung (B) eingesetzt.

### Beispiele

Es wurden die folgenden Zusammensetzungen (A) hergestellt (alle Angaben in Gew.-%)

| | Bsp 1 | Bsp 2 | Bsp. 3 |
|---|---|---|---|
| Ceteareth-50 | 3,70 | 3,70 | 3,70 |
| Ceteareth-2 | 0,24 | 0,24 | 0,24 |
| Glycerylmonostearat | 0,50 | 0,50 | 0,50 |
| Cetearylalkohol | 11,0 | 11,0 | 11,0 |
| Ammoniak (25 %ige wässrige Lösung) | 6,00 | 6,00 | 6,00 |
| Titandioxid | 0,5 | 0,5 | 0,5 |
| Monoethanolamin | 0,6 | 0,6 | 0,6 |
| Natriumsulfit | 0,35 | 0,35 | 0,35 |
| Linoleamidopropyl PG-Dimoniumchlorid, Phosphat | 0,03 | 0,03 | 0,03 |
| EDTA (Dinatriumsalz) | 0,2 | 0,2 | 0,2 |
| POlyquaternium-39 (Dimethyld iallylammon i u m chloride, acrylamide, acrylic acid terpolymer) | 0,15 | 0,15 | 0,15 |
| Vitamin C | 0,1 | 0,1 | 0,1 |
| p-Toluylendiamin, Sulfat | 1,178 | 0,011 | 0,22 |
| Resorcin | 0,275 | --- | 0,03 |
| 2-Methylresorcin | 0,266 | --- | 0,1 |
| m-Aminophenol | 0,0378 | --- | --- |
| 2-Amino-3-hydroxypyridin | 0,038 | --- | 0,19 |
| 2-Amino-4-Hydroxyethylamino-anisol, Sulfat | --- | 0,01 | |
| 5-Amino-2-methylphenol | --- | 0,001 | 0,45 |
| 4-Amino-3-methylphenol | --- | --- | 0,37 |
| Wasser | ad 100 | ad 100 | ad 100 |
| Viskosität (20 °C / Haake Rheostress 6000 / gemessen mit Cone 35/1 Geometrie, Durchmesser 35 mm und 1° Winkel / Scherrate 7,2 s⁻¹) 2 months after production | 20094 mPas | 20161 mPas | 19204 mPas |

| | Bsp 4 | Bsp 5 | Bsp 6 |
|---|---|---|---|
| Ceteareth-50 | 3,70 | 3,70 | 3,70 |
| Ceteareth-2 | 0,24 | 0,24 | 0,24 |
| Glyceryl-monostearat | 0,50 | 0,50 | 0,50 |
| Cetearylalkohol | 11,0 | 11,0 | 11,0 |
| Ammoniak (25 %ige wässrige Lösung) | 6,00 | 6,00 | 6,00 |
| Titandioxid | 0,5 | 0,5 | 0,5 |
| Monoethanolamin | 0,6 | 0,6 | 0,6 |
| Natriumsulfit | 0,35 | 0,35 | 0,35 |
| Linoleamidopropyl PG-Dimoniumchlorid, Phosphat | 0,03 | 0,03 | 0,03 |
| EDTA (Dinatriumsalz) | 0,2 | 0,2 | 0,2 |
| POlyquaternium-39 (Dimethyld iallylammon i u m chloride, acrylamide, acrylic acid terpolymer) | 0,15 | 0,15 | 0,15 |
| Vitamin C | 0,1 | 0,1 | 0,1 |
| p-Toluylendiamin, Sulfat | 0,85 | 2,55 | 0,295 |
| Resorcin | 0,26 | 0,90 | 0,13 |
| 2-Methylresorcin | --- | | 0,05 |
| m-Aminophenol | 0,10 | 0,33 | 0,01 |
| 2-Amino-3-hydroxypyridin | --- | --- | --- |
| 2-Amino-4-Hydroxyethylamino-anisol, Sulfat | --- | 0,03 | --- |
| 5-Amino-2-methylphenol | --- | --- | --- |
| 4-Chlorresorcin | 9,13 | --- | --- |
| 2,4-Diaminophenoxyethanol, Dihydrochlorid | --- | --- | --- |
| 4-Amino-3-methylphenol | --- | --- | 0,05 |
| Wasser | ad 100 | ad 100 | ad 100 |
| Viskosität (20 °C / Haake Rheostress 6000 / gemessen mit Cone 35/1 Geometrie, Durchmesser 35 mm und 1° Winkel / Scherrate 7,2 s⁻¹) 2 month after production | 20897 mPas | 19234 mPas | 20584 mPas |

## Patentansprüche

1. Mehrkomponenten-Verpackungseinheit (Kit-of-Parts) zum oxidativen Färben von keratinischen Fasern, insbesondere menschlichen Haaren, umfassend getrennt voneinander konfektioniert
- einen Container (I) enthaltend ein kosmetisches Mittel (A) und
- einen Container (II) enthaltend ein kosmetisches Mittel (B),
wobei
- das Mittel (A) in Container (I) ein Mittel zur Färbung von keratinischen Fasern ist, enthaltend in einem wässrigen Träger
(a) einen oder mehrere hoch ethoxylierte C₁₆-C₁₈-Fettalkohole mit 30 bis 100 Ethoxy-Gruppen,
(b) einen oder mehrere niedrig ethoxylierte C₁₆-C₁₈-Fettalkohole mit 1 bis 5 Ethoxygruppen,
(c) einen oder mehrere Monoester aus Glycerin und einer C₁₄-C₂₀-Fettsäure,
(d) einen oder mehrere C₁₆-C₁₈-Fettalkohole,
(e) ein oder mehrere Alkalisierungsmittel und
(f) ein oder mehrere Oxidationsfarbstoffvorprodukte,
wobei
- das Gewichtsverhältnis aus allen im Mittel enthaltenen hoch ethoxylierten C₁₆-C₁₈-Fettalkoholen (a) zu allen im Mittel enthaltenen niedrig ethoxylierten C₁₆-C₁₈-Fettalkoholen (b), d.h. das Gewichtsverhältnis (a)/(b), bei einem Wert von 10 bis 20 liegt und
- das Gewichtsverhältnis aus der Summe aller im Mittel enthaltenen Bestandteile [(c)+(d)] zur Summe aller im Mittel enthaltenden ethoxylierten Fettalkohole [(a)+(b)], d.h. das Gewichtsverhältnis [(c)+(d)]/[(a)+(b)], bei einem Wert von 2 bis 5 liegt und
- das Mittel (B) in Container (II) eine Oxidationsmittelzubereitung (B) ist, die Wasserstoffperoxid enthält.

2. Mehrkomponenten-Verpackungseinheit nach Anspruch 1, **dadurch gekennzeichnet, dass** das Mittel (A) (a) einen oder mehrere hoch ethoxylierte C₁₆-C₁₈-Fettalkohole der Formel (I) enthält, 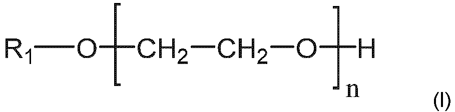 worin
R1 für eine lineare oder verzweigte, gesättigte oder ungesättigte C₁₆-C₁₈-Alkylgruppe steht und
n für eine ganze Zahl von 30 bis 100, bevorzugt von 35 bis 90, weiter bevorzugt von 40 bis 80 und ganz besonders bevorzugt von 45 bis 70 steht.

3. Mehrkomponenten-Verpackungseinheit nach einem der Ansprüche 1 bis 2, **dadurch gekennzeichnet, dass** das Mittel (A) - bezogen auf das Gesamtgewicht des Mittels (A) -
(a) einen oder mehrere hoch ethoxylierte C₁₆-C₁₈-Fettalkohole mit 30 bis 100 Ethoxy-Gruppen in einer Gesamtmenge von 2,0 bis 10,0 Gew.-%, bevorzugt von 2,0 bis 8,0 Gew.-%, weiter bevorzugt von 2,0 bis 6,0 Gew.-% und ganz besonders bevorzugt von 3,0 bis 5,0 Gew.-% enthält.

4. Mehrkomponenten-Verpackungseinheit nach einem der Ansprüche 1 bis 3, **dadurch gekennzeichnet, dass** das Mittel (A) (b) einen oder mehrere niedrig ethoxylierte C₁₆-C₁₈-Fettalkohole der Formel (II) enthält, 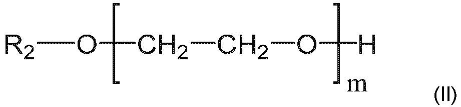 worin
R2 für eine lineare oder verzweigte, gesättigte oder ungesättigte C₁₆-C₁₈-Alkylgruppe steht und
m für eine ganze Zahl von 1 bis 5, bevorzugt von 1 bis 4 und ganz besonders bevorzugt von 1 bis 3 steht.

5. Mehrkomponenten-Verpackungseinheit nach einem der Ansprüche 1 bis 4, **dadurch gekennzeichnet, dass** das Mittel (A) - bezogen auf das Gesamtgewicht des Mittels (A) -
(b) einen oder mehrere niedrig ethoxylierte C₁₆-C₁₈-Fettalkohole mit 1 bis 5 Ethoxy-Gruppen in einer Gesamtmenge von 0,05 bis 1,5 Gew.-%, bevorzugt von 0,05 bis 1,25 Gew.-%, weiter bevorzugt von 0,1 bis 1,0 Gew.-% und ganz besonders bevorzugt von 0,1 bis 0,75 Gew.-% enthält.

6. Mehrkomponenten-Verpackungseinheit nach einem der Ansprüche 1 bis 5, **dadurch gekennzeichnet, dass** das Gewichtsverhältnis aus allen im Mittel (A) enthaltenen hoch ethoxylierten C₁₆-C₁₈-Fettalkoholen (a) zu allen im Mittel (A) enthaltenen niedrig ethoxylierten C₁₆-C₁₈-Fettalkoholen (b), d.h. das Gewichtsverhältnis (a)/(b), bei einem Wert von 11 bis 19, bevorzugt von 12 bis 18, weiter bevorzugt von 13 bis 17 und ganz besonders bevorzugt von 14 bis 16 liegt.

7. Mehrkomponenten-Verpackungseinheit nach einem der Ansprüche 1 bis 6, **dadurch gekennzeichnet, dass** das Mittel (A) (c) einen oder mehrere Monoester aus Glycerin und einer C₁₄-C₂₀-Fettsäure der Formel (III) enthält, 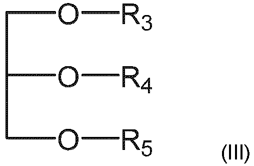 worin
R3, R4, R5 unabhängig voneinander für ein Wasserstoffatom oder eine Acylgruppe -C(O)R₆ stehen, in der R₆ für eine unverzweigte oder verzweigte, gesättigte oder ungesättigte C₁₃-C₁₉-Alkylgruppe, bevorzugt eine unverzweigte, gesättigte C₁₅-C₁₇-Alkylgruppe, steht, mit der Maßgabe, dass zwei der Reste aus R3, R4 und R5 für ein Wasserstoffatom stehen und der dritte Rest für eine Acylgruppe - C(O)R₆ steht.

8. Mehrkomponenten-Verpackungseinheit nach einem der Ansprüche 1 bis 7, **dadurch gekennzeichnet, dass** das Mittel (A) - bezogen auf das Gesamtgewicht des Mittels (A) -
(c) einen oder mehrere Monoester aus Glycerin und einer C₁₄-C₂₀-Fettsäure in einer Gesamtmenge von 0,1 bis 5,0 Gew.-%, bevorzugt von 0, 1 bis 2,5 Gew.-%, weiter bevorzugt von 0,1 bis 1,3 Gew.-% und ganz besonders bevorzugt von 0,2 bis 0,7 Gew.-% enthält.

9. Mehrkomponenten-Verpackungseinheit nach einem der Ansprüche 1 bis 8, **dadurch gekennzeichnet, dass** das Mittel (A) - bezogen auf das Gesamtgewicht des Mittels (A) -
(d) einen oder mehrere C₁₆-C₁₈-Fettalkohole in einer Gesamtmenge von 0,1 bis 20,0 Gew.-%, bevorzugt von 5,0 bis 15,0 Gew.-5, weiter bevorzugt von 10,0 bis 13,0 Gew.-% und ganz besonders bevorzugt von 10,5 bis 12,5 Gew.-% enthält.

10. Mehrkomponenten-Verpackungseinheit nach einem der Ansprüche 1 bis 9, **dadurch gekennzeichnet, dass** das Gewichtsverhältnis aus der Summe aller im Mittel (A) enthaltenen Bestandteile [(c)+(d)] zur Summe aller im Mittel (A) enthaltenden ethoxylierten Fettalkohole [(a)+(b)], d.h. das Gewichtsverhältnis [(c)+(d)]/[(a)+(b)] bei einem Wert von 2,0 bis 4,5, bevorzugt von 2,0 bis 4,0, weiter bevorzugt von 2,0 bis 3,5 und ganz besonders bevorzugt von 2,5 bis 3,0 liegt.

11. Mehrkomponenten-Verpackungseinheit nach einem der Ansprüche 1 bis 10, **dadurch gekennzeichnet, dass** das Mittel (A) ein oder mehrere Alkalisierungsmittel (e) aus der Gruppe aus Ammoniak, 2-Aminoethan-1-ol, 2-Amino-2-methylpropan-1-ol, 2-Amino-2-methyl-propan-1,3-diol , L-Arginin, D-Arginin, D/L-Arginin, L-Lysin, D-Lysin und D/L-Lysin enthält.

12. Mehrkomponenten-Verpackungseinheit nach einem der Ansprüche 1 bis 11, **dadurch gekennzeichnet, dass** das Mittel (A) ein oder mehrere Oxidationsfarbstoffvorprodukte (f) aus der Gruppe aus Phenylendiamin Sulfat, Phenylendiamin Monohydrochlorid, Phenylendiamin Dihydrochlorid, p-Toluylendiamin Sulfat, p-Toluylendiamin Monohydrochlorid, p-Toluylendiamin Dihydrochlorid, 2-(2-Hydroxyethyl)-p-phenylendiamin Sulfat, 2-(2-Hydroxyethyl)-p-phenylendiamin Monohydrochlorid, 2-(2-Hydroxyethyl)-p-phenylendiamin Dihydrochlorid, N,N-Bis-(2-hydroxyethyl)-p-phenylendiamin Sulfat, N,N-Bis-(2-hydroxyethyl)-p-phenylendiamin Monohydrochlorid, N,N-Bis-(2-hydroxyethyl)-p-phenylendiamin Dihydrochlorid, 2-Methoxymethyl-p-phenylendiamin Sulfat, 2-Methoxymethyl-p-phenylendiamin Monohydrochlorid, 2-Methoxymethyl-p-phenylendiamin Dihydrochlorid, p-Aminophenol Hydrogensulfat, p-Aminophenol Monohydrochlorid, 4-Amino-3-methylphenol Hydrogensulfat, 4-Amino-3-methylphenol Chlorid, 2,4,5,6-Tetraaminopyrimidin Monosulfat, 2,4,5,6-Tetraaminopyrimidin Disulfat, 2,4,5,6-Tetraaminopyrimidin Monohydrochlorid, 2,4,5,6-Tetraaminopyrimidin Dihydrochlorid, 2,4,5,6-Tetraaminopyrimidin Trihydrochlorid, 2,4,5,6-Tetraaminopyrimidin Tetrahydrochlorid, 4-Hydroxy-2,5,6-triaminopyrimidin Sulfat, 4-Hydroxy-2,5,6-triaminopyrimidin Monohydrochlorid, 4-Hydroxy-2,5,6-triaminopyrimidin Diydrochlorid, 4-Hydroxy-2,5,6-triaminopyrimidin Trihydrochlorid, 4,5-Diamino-1-(2-hydroxyethyl)-1H-pyrazol Sulfat, 4,5-Diamino-1-(2-hydroxyethyl)-1H-pyrazol Monohydrochlorid und/oder 4,5-Diamino-1-(2-hydroxyethyl)-1H-pyrazol Dihydrochlorid enthält.

13. Mehrkomponenten-Verpackungseinheit nach einem der Ansprüche 1 bis 12, **dadurch gekennzeichnet, dass** das Mittel (A) - bezogen auf das Gesamtgewicht des Mittels (A) - ein oder mehrere Oxidationsfarbstoffvorprodukte (f) aus der Gruppe der Sulfate, Chloride und Bromide in einer Gesamtmenge von 0,01 bis 5,5 Gew.-%, bevorzugt von 0,7 bis 5,0 Gew.-%, weiter bevorzugt von 0,9 bis 4,5 Gew.-% und ganz besonders bevorzugt von 2,1 bis 3,4 Gew.-% enthält.

14. Mehrkomponenten-Verpackungseinheit nach einem der Ansprüche 1 bis 13, **dadurch gekennzeichnet, dass** das Mittel (A) eine Viskosität von 10.000 bis 30.000 mPas, bevorzugt von 12.000 bis 25.000 mPas und ganz besonders bevorzugt von 16.000 bis 21.000 mPas besitzt (20 °C / Haake Rheostress 6000 / gemessen mit Cone 35/1 Geometrie, Durchmesser 35 mm und 1° Winkel / Scherrate 7,2 s⁻¹).

15. Mehrkomponenten-Verpackungseinheit nach einem der Ansprüche 1 bis 14, **dadurch gekennzeichnet, dass** das Mittel (A) - bezogen auf das Gesamtgewicht des Mittels (A) - 0,1 bis 10 Gew.-%, bevorzugt von 0,5 bis 5,0 Gew.-% und ganz besonders bevorzugt von 1,0 bis 3,0 Gew.-% Octyldodecanol enthält.

## Claims

1. A multi-component packaging unit (kit-of-parts) for oxidative dyeing of keratin fibers, in particular human hair, comprising, packaged separately from one another,
- a container (I) containing a cosmetic agent (A) and
- a container (II) containing a cosmetic agent (B),
wherein
- the agent (A) in container (I) is an agent for dyeing keratin fibers, contained in an aqueous carrier
(a) one or more highly ethoxylated C₁₆-C₁₈ fatty alcohols having 30 to 100 ethoxy groups,
(b) one or more low ethoxylated C₁₆-C₁₈ fatty alcohols having 1 to 5 ethoxy groups,
(c) one or more monoesters of glycerol and a C₁₄-C₂₀ fatty acid,
(d) one or more C₁₆-C₁₈ fatty alcohols,
(e) one or more alkalizing agents and
(f) one or more oxidation dye precursors,
wherein
- the weight ratio of all highly ethoxylated C₁₆-C₁₈ fatty alcohols (a) contained in the agent to all low ethoxylated C₁₆-C₁₈ fatty alcohols (b) contained in the agent, i.e. the weight ratio (a)/(b), has a value from 10 to 20 and
- the weight ratio of the sum of all components [(c)+(d)] contained in the agent to the sum of all ethoxylated fatty alcohols [(a)+(b)] contained in the agent, i.e. the weight ratio [(c)+(d)]/[(a)+(b)], has a value from 2 to 5 and
- the agent (B) in container (II) is an oxidizing agent preparation (B) containing hydrogen peroxide.

2. Multi-component packaging unit according to claim 1, **characterized in that** the agent (A) (a) contains one or more highly ethoxylated C₁₆-C₁₈ fatty alcohols of formula (I), 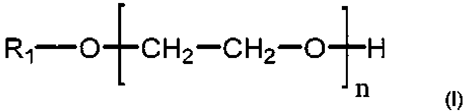 where
R1 represents a linear or branched, saturated or unsaturated C₁₆-C₁₈ alkyl group and
n represents an integer from 30 to 100, preferably from 35 to 90, more preferably from 40 to 80, and very particularly preferably from 45 to 70.

3. Multi-component packaging unit according to one of claims 1 to 2, **characterized in that** the agent (A) contains - based on the total weight of the agent (A) -
(a) one or more highly ethoxylated C₁₆-C₁₈ fatty alcohols having 30 to 100 ethoxy groups in a total amount of from 2.0 to 10.0 wt.%, preferably from 2.0 to 8.0 wt.%, more preferably from 2.0 to 6.0 wt.%, and very particularly preferably from 3.0 to 5.0 wt.%.

4. Multi-component packaging unit according to one of claims 1 to 3, **characterized in that** the agent (A) (b) contains one or more low ethoxylated C₁₆-C₁₈ fatty alcohols of formula (II), 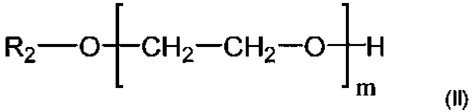 where
R2 represents a linear or branched, saturated or unsaturated C₁₆-C₁₈ alkyl group and
m represents an integer from 1 to 5, preferably 1 to 4, and very particularly preferably 1 to 3.

5. Multi-component packaging unit according to one of claims 1 to 4, **characterized in that** the agent (A) contains - based on the total weight of the agent (A) -
(b) one or more low ethoxylated C₁₆-C₁₈ fatty alcohols having 1 to 5 ethoxy groups in a total amount of from 0.05 to 1.5 wt.%, preferably from 0.05 to 1.25 wt.%, more preferably from 0.1 to 1.0 wt.%, and very particularly preferably from 0.1 to 0.75 wt.%.

6. Multi-component packaging unit according to one of claims 1 to 5, **characterized in that** the weight ratio of all the highly ethoxylated C₁₆-C₁₈ fatty alcohols (a) contained in the agent (A) to all the low ethoxylated C₁₆-C₁₈ fatty alcohols (b) contained in the agent (A), i.e. the weight ratio (a)/(b), has a value from 11 to 19, preferably from 12 to 18, more preferably from 13 to 17, and very particularly preferably from 14 to 16.

7. Multi-component packaging unit according to one of claims 1 to 6, **characterized in that** the agent (A) (c) contains one or more monoesters of glycerol and a C₁₄-C₂₀ fatty acid of formula (III), 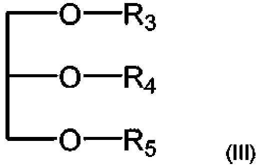 where
R3, R4, R5 independently represent a hydrogen atom or an acyl group -C(O)R₆, in which R₆ represents an unbranched or branched, saturated or unsaturated C₁₃-C₁₉ alkyl group, preferably an unbranched, saturated C₁₅-C₁₇ alkyl group, with the proviso that two of the groups of R3, R4 and R5 represent hydrogen and the third group represents an acyl group -C(O)R₆.

8. Multi-component packaging unit according to one of claims 1 to 7, **characterized in that** the agent (A) contains - based on the total weight of the agent (A) -
(c) one or more monoesters of glycerol and a C₁₄-C₂₀ fatty acid in a total amount of from 0.1 to 5.0 wt.%, preferably from 0.1 to 2.5 wt.%, more preferably from 0.1 to 1.3 wt.%, and very particularly preferably from 0.2 to 0.7 wt.%.

9. Multi-component packaging unit according to one of claims 1 to 8, **characterized in that** the agent (A) contains - based on the total weight of the agent (A) -
(d) one or more C₁₆-C₁₈ fatty alcohols in a total amount of from 0.1 to 20.0 wt.%, preferably from 5.0 to 15.0 wt.%, more preferably from 10.0 to 13.0 wt.%, and very particularly preferably from 10.5 to 12.5 wt.%.

10. Multi-component packaging unit according to one of claims 1 to 9, **characterized in that** the weight ratio of the sum of all components [(c)+(d)] contained in the agent (A) to the sum of all ethoxylated fatty alcohols [(a)+(b)] contained in the agent (A), i.e. the weight ratio [(c)+(d)]/[(a)+(b)] has a value of from 2.0 to 4.5, preferably from 2.0 to 4.0, more preferably from 2.0 to 3.5, and very particularly preferably from 2.5 to 3.0.

11. Multi-component packaging unit according to one of claims 1 to 10, **characterized in that** the agent (A) contains one or more alkalizing agents (e) from the group consisting of ammonia, 2-aminoethan-1-ol, 2-amino-2-methylpropan-1-ol, 2-amino-2-methylpropane-1,3-diol, L-arginine, D-arginine, D/L-arginine, L-lysine, D-lysine and D/L-lysine.

12. Multi-component packaging unit according to one of claims 1 to 11, **characterized in that** the agent (A) contains one or more oxidation dye precursors (f) from the group consisting of phenylenediamine sulfate, phenylenediamine monohydrochloride, phenylenediamine dihydrochloride, p-toluenediamine sulfate, p-toluenediamine monohydrochloride, p-toluenediamine dihydrochloride, 2-(2-hydroxyethyl)-p-phenylenediamine sulfate, 2-(2-hydroxyethyl)-p-phenylenediamine monohydrochloride, 2-(2-hydroxyethyl)-p-phenylenediamine dihydrochloride, N,N-bis-(2-hydroxyethyl)-p-phenylenediamine sulfate, N,N-bis-(2-hydroxyethyl)-p-phenylenediamine monohydrochloride, N,N-bis-(2-hydroxyethyl)-p-phenylenediamine dihydrochloride, 2-methoxymethyl-p-phenylenediamine sulfate, 2-methoxymethyl-p-phenylenediamine monohydrochloride, 2-methoxymethyl-p-phenylenediamine dihydrochloride, p-aminophenol hydrogen sulfate, p-aminophenol monohydrochloride, 4-amino-3-methylphenol hydrogen sulfate, 4-amino-3-methylphenol chloride, 2,4,5,6-tetraaminopyrimidine monosulfate, 2,4,5,6-tetraaminopyrimidine disulfate, 2,4,5,6-tetraaminopyrimidine monohydrochloride, 2,4,5,6-tetraaminopyrimidine dihydrochloride, 2,4,5,6-tetraaminopyrimidine trihydrochloride, 2,4,5,6-tetraaminopyrimidine tetrahydrochloride, 4-hydroxy-2,5,6-triaminopyrimidine sulfate, 4-hydroxy-2,5,6-triaminopyrimidine monohydrochloride, 4-hydroxy-2,5,6-triaminopyrimidine dihydrochloride, 4-hydroxy-2,5,6-triaminopyrimidine trihydrochloride, 4,5-diamino-1-(2-hydroxyethyl)-1H-pyrazole sulfate, 4,5-diamino-1-(2-hydroxyethyl)-1H-pyrazole monohydrochloride and/or 4,5-diamino-1-(2-hydroxyethyl)-1H-pyrazole dihydrochloride.

13. Multi-component packaging unit according to one of claims 1 to 12, **characterized in that** the agent (A) contains - based on the total weight of the agent (A) - one or more oxidation dye precursors (f) from the group of sulfates, chlorides and bromides in a total amount of 0.01 to 5.5 wt.%, preferably 0.7 to 5.0 wt.%, more preferably 0.9 to 4.5 wt.%, and very particularly preferably 2.1 to 3.4 wt.%.

14. Multi-component packaging unit according to one of claims 1 to 13, **characterized in that** the agent (A) has a viscosity of 10,000 to 30,000 mPas, preferably 12,000 to 25,000 mPas and very particularly preferably 16,000 to 21,000 mPas (20 °C / Haake Rheostress 6000 / measured with cone 35/1 geometry, diameter 35 mm and 1° angle/shear rate 7.2 s⁻¹).

15. Multi-component packaging unit according to one of claims 1 to 14, **characterized in that** the agent (A) contains - based on the total weight of the agent (A) - 0.1 to 10 wt.%, preferably from 0.5 to 5.0 wt.% and very particularly preferably from 1.0 to 3.0 wt.% of octyldodecanol.

## Revendications

1. Unité de conditionnement multi-composants (kit) pour la coloration par oxydation de fibres kératiniques, en particulier de cheveux humains, comprenant, conditionnés séparément les uns des autres,
- un récipient (I) contenant un agent cosmétique (A), et
- un récipient (II) contenant un agent cosmétique (B),
dans laquelle
- l'agent (A) dans le récipient (I) est un agent permettant la coloration de fibres kératiniques, contenant dans un véhicule aqueux
(a) un ou plusieurs alcools gras en C₁₆-C₁₈ fortement éthoxylés comportant 30 à 100 groupes éthoxy,
(b) un ou plusieurs alcools gras en C₁₆-C₁₈ faiblement éthoxylés comportant 1 à 5 groupes éthoxy,
(c) un ou plusieurs monoesters de glycérol et d'un acide gras en C₁₄-C₂₀,
(d) un ou plusieurs alcools gras en C₁₆-C₁₈,
(e) un ou plusieurs agents d'alcalinisation, et
(f) un ou plusieurs précurseurs de colorant d'oxydation,
dans laquelle
- le rapport pondéral de tous les alcools gras en d₁₆-C₁₈ fortement éthoxylés contenus dans l'agent (a) à tous les alcools gras en d₁₆-C₁₈ faiblement éthoxylés contenus dans l'agent (b), c'est-à-dire le rapport pondéral (a)/(b), est une valeur de 10 à 20, et
- le rapport pondéral de la somme de tous les composants contenus dans l'agent [(c)+(d)] à la somme de tous les alcools gras éthoxylés contenus dans l'agent [(a)+(b)], c'est-à-dire le rapport pondéral [(c)+(d)]/[(a)+(b)], est une valeur de 2 à 5, et
- l'agent (B) dans le récipient (II) est une préparation d'agent d'oxydation (B) contenant du peroxyde d'hydrogène.

2. Unité de conditionnement multi-composants selon la revendication 1, **caractérisée en ce que** l'agent (A) contient (a) un ou plusieurs alcools gras en C₁₆-C₁₈ fortement éthoxylés de formule (I), 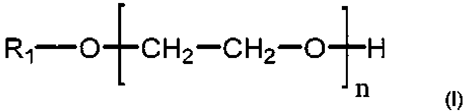 où
R1 représente un groupe alkyle en d₁₆-C₁₈, linéaire ou ramifié, saturé ou insaturé, et
n représente un nombre entier de 30 à 100, de préférence de 35 à 90, plus préférablement de 40 à 80 et de manière particulièrement préférée de 45 à 70.

3. Unité de conditionnement multi-composants selon l'une des revendications 1 à 2, **caractérisée en ce que** l'agent (A) contient, par rapport au poids total de l'agent (A),
(a) un ou plusieurs alcools gras en C₁₆-C₁₈ fortement éthoxylés comportant 30 à 100 groupes éthoxy en une quantité totale de 2,0 à 10,0 % en poids, de préférence de 2,0 à 8,0 % en poids, plus préférablement de 2,0 à 6,0 % en poids et de manière particulièrement préférée de 3,0 à 5,0 % en poids.

4. Unité de conditionnement multi-composants selon l'une des revendications 1 à 3, **caractérisée en ce que** l'agent (A) contient (b) un ou plusieurs alcools gras en C₁₆-C₁₈ faiblement éthoxylés de formule (II), 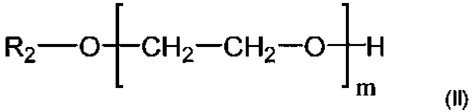 où
R2 représente un groupe alkyle en d₁₆-C₁₈, linéaire ou ramifié, saturé ou insaturé, et
m représente un nombre entier de 1 à 5, de préférence de 1 à 4, et de manière particulièrement préférée de 1 à 3.

5. Unité de conditionnement multi-composants selon l'une des revendications 1 à 4, **caractérisée en ce que** l'agent (A) contient, par rapport au poids total de l'agent (A),
(b) un ou plusieurs alcools gras en C₁₆-C₁₈ faiblement éthoxylés comportant 1 à 5 groupes éthoxy en une quantité totale de 0,05 à 1,5 % en poids, de préférence de 0,05 à 1,25 % en poids, plus préférablement de 0,1 à 1,0 % en poids et de manière particulièrement préférée de 0,1 à 0,75 % en poids.

6. Unité de conditionnement multi-composants selon l'une des revendications 1 à 5, **caractérisée en ce que** le rapport pondéral de tous les alcools gras en d₁₆-C₁₈ fortement éthoxylés (a) contenus dans l'agent (A) à tous les alcools gras en d₁₆-C₁₈ faiblement éthoxylés (b) contenus dans l'agent (A), c'est-à-dire le rapport pondéral (a)/(b), est une valeur de 11 à 19, de préférence de 12 à 18, plus préférablement de 13 à 17 et de manière particulièrement préférée de 14 à 16.

7. Unité de conditionnement multi-composants selon l'une des revendications 1 à 6, **caractérisée en ce que** l'agent (A) contient (c) un ou plusieurs monoesters de glycérol et d'un acide gras en C₁₄-C₂₀ de formule (III), 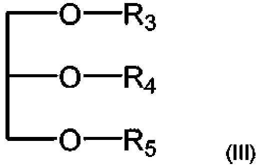 où
R3, R4, R5 représentent indépendamment les uns des autres un atome d'hydrogène ou un groupe acyle -C(O)R₆, dans lequel R₆ représente un groupe alkyle en C₁₃-C₁₉, non ramifié ou ramifié, saturé ou insaturé, de préférence un groupe alkyle en C₁₅-C₁₇ saturé et non ramifié, à condition que deux des radicaux parmi R3, R4 et R5 représentent un atome d'hydrogène et que le troisième radical représente un groupe acyle -C(O)R₆.

8. Unité de conditionnement multi-composants selon l'une des revendications 1 à 7, **caractérisée en ce que** l'agent (A) contient, par rapport au poids total de l'agent (A),
(c) un ou plusieurs monoesters de glycérol et d'un acide gras en C₁₄-C₂₀ en une quantité totale de 0,1 à 5,0 % en poids, de préférence de 0,1 à 2,5 % en poids, plus préférablement de 0,1 à 1,3 % en poids et de manière particulièrement préférée de 0,2 à 0,7 % en poids.

9. Unité de conditionnement multi-composants selon l'une des revendications 1 à 8, **caractérisée en ce que** l'agent (A) contient, par rapport au poids total de l'agent (A),
(d) un ou plusieurs alcools gras en C₁₆-C₁₈ en une quantité totale de 0,1 à 20,0 % en poids, de préférence de 5,0 à 15,0 % en poids, plus préférablement de 10,0 à 13,0 % en poids et de manière particulièrement préférée de 10,5 à 12,5 % en poids.

10. Unité de conditionnement multi-composants selon l'une des revendications 1 à 9, **caractérisée en ce que** le rapport pondéral de la somme de tous les composants contenus dans l'agent (A) [(c)+(d)] à la somme de tous les alcools gras éthoxylés contenus dans l'agent (A) [(a)+(b)], c'est-à-dire le rapport pondéral [(c)+(d)]/[(a)+(b)], est une valeur de 2,0 à 4,5, de préférence de 2,0 à 4,0, plus préférablement de 2,0 à 3,5 et de manière particulièrement préférée de 2,5 à 3,0.

11. Unité de conditionnement multi-composants selon l'une des revendications 1 à 10, **caractérisée en ce que** l'agent (A) contient un ou plusieurs agents d'alcalinisation (e) choisis dans le groupe constitué par l'ammoniac, le 2-aminoéthan-1-ol, le 2-amino-2-méthylpropan-1-ol, le 2-amino-2-méthyl-propane-1,3-diol, la L-arginine, la D-arginine, la D/L-arginine, la L-lysine, la D-lysine et la D/L-lysine.

12. Unité de conditionnement multi-composants selon l'une des revendications 1 à 11, **caractérisée en ce que** l'agent (A) contient un ou plusieurs précurseurs de colorant d'oxydation (f) choisis dans le groupe constitué par le sulfate de phénylènediamine, le monochlorhydrate de phénylènediamine, le dichlorhydrate de phénylènediamine, le sulfate de p-toluylènediamine, le monochlorhydrate de p-toluylènediamine, le dichlorhydrate de p-toluylènediamine, le sulfate de 2-(2-hydroxyéthyl)-p-phénylènediamine, le monochlorhydrate de 2-(2-hydroxyéthyl)-p-phénylènediamine, le dichlorhydrate de 2-(2-hydroxyéthyl)-p-phénylènediamine, le sulfate de N,N-bis-(2-hydroxyéthyl)-p-phénylènediamine, le monochlorhydrate de N,N-bis-(2-hydroxyéthyl)-p-phénylènediamine, le dichlorhydrate de N,N-bis-(2-hydroxyéthyl)-p-phénylènediamine, le sulfate de 2-méthoxyméthyl-p-phénylènediamine, le monochlorhydrate de 2-méthoxyméthyl-p-phénylènediamine, le dichlorhydrate de 2-méthoxyméthyl-p-phénylènediamine, l'hydrogénosulfate de p-aminophénol, le monochlorhydrate de p-aminophénol, l'hydrogénosulfate de 4-amino-3-méthylphénol, le chlorure de 4-amino-3-méthylphénol, le monosulfate de 2,4,5,6-tétraaminopyrimidine, le disulfate de 2,4,5,6-tétraaminopyrimidine, le monochlorhydrate de 2,4,5,6-tétraaminopyrimidine, le dichlorhydrate de 2,4,5,6-tétraaminopyrimidine, le trichlorhydrate de 2,4,5,6-tétraaminopyrimidine, le tétrachlorhydrate de 2,4,5,6-tétraaminopyrimidine, le sulfate de 4-hydroxy-2,5,6-triaminopyrimidine, le monochlorhydrate de 4-hydroxy-2,5,6-triaminopyrimidine, le dichlorhydrate de 4-hydroxy-2,5,6-triaminopyrimidine, le trichlorhydrate de 4-hydroxy-2,5,6-triaminopyrimidine, le sulfate de 4,5-diamino-1-(2-hydroxyéthyl)-1H-pyrazole, le monochlorhydrate de 4,5-diamino-1-(2-hydroxyéthyl)-1H-pyrazole et/ou le dichlorhydrate de 4,5-diamino-1-(2-hydroxyéthyl)-1H-pyrazole.

13. Unité de conditionnement multi-composants selon l'une des revendications 1 à 12, **caractérisée en ce que** l'agent (A) contient, par rapport au poids total de l'agent (A), un ou plusieurs précurseurs de colorant d'oxydation (f) choisis dans le groupe constitué par les sulfates, les chlorures et les bromures, en une quantité totale de 0,01 à 5,5 % en poids, de préférence de 0,7 à 5,0 % en poids, plus préférablement de 0,9 à 4,5 % en poids et de manière particulièrement préférée de 2,1 à 3,4 % en poids.

14. Unité de conditionnement multi-composants selon l'une des revendications 1 à 13, **caractérisée en ce que** l'agent (A) présente une viscosité de 10 000 à 30 000 mPas, de préférence de 12 000 à 25 000 mPas, et de manière particulièrement préférée de 16 000 à 21 000 mPas (20 °C/Haake Rheostress 6000/mesurée avec une géométrie cône 35/1, un diamètre de 35 mm et un angle de 1°/une vitesse de cisaillement de 7,2 s⁻¹).

15. Unité de conditionnement multi-composants selon l'une des revendications 1 à 14, **caractérisée en ce que** l'agent (A), par rapport au poids total de l'agent (A), contient de 0,1 à 10 % en poids, de préférence de 0,5 à 5,0 % en poids et de manière particulièrement préférée de 1,0 à 3,0 % en poids d'octyldodécanol.
